(19) Europäisches Patentamt
European Patent Office
Office européen des brevets

(11) Publication number: 0 199 480 B1

(12) # EUROPEAN PATENT SPECIFICATION

(45) Date of publication of patent specification: 01.07.92

(51) Int. Cl.5: C12N 15/41, A61K 39/29, C12P 21/02

(21) Application number: 86302465.9

(22) Date of filing: 03.04.86

(54) **Hepatitis A virus vaccines.**

(30) Priority: 03.04.85 US 719329

(43) Date of publication of application:
29.10.86 Bulletin 86/44

(45) Publication of the grant of the patent:
01.07.92 Bulletin 92/27

(84) Designated Contracting States:
AT BE CH DE FR GB IT LI LU NL SE

(56) References cited:
EP-A- 0 061 740
EP-A- 0 154 587
EP-A- 0 175 613
WO-A-85/01517
US-A- 4 031 203
US-A- 4 164 566

PROC. NATL. ACAD. SCI. USA, vol. 80, October 1983, pages 5885-5889, Baltimore, US; J.R. TICEHURST et al.: "Molecular cloning and characterization of hepatitis A virus cDNA"

PROC. NATL. ACAD. SCI. USA, vol. 82, May 1985, pages 2627-2631, Baltimore, US; R. NAJARIAN et al.: "Primary structure and gene organization of human hepatitis A virus"

(73) Proprietor: **CHIRON CORPORATION**
**4560 Horton Street**
**Emeryville, California 94608(US)**

(72) Inventor: **Dina, Dino**
**1254 Washington Street**
**San Francisco California 94108(US)**
Inventor: **Potter, Steven J.**
**132 Ross Valley Drive**
**San Rafael California 94901(US)**
Inventor: **van Nest, Gary A.**
**4898 San Pablo Dam Road**
**El Sobrante California 94803(US)**
Inventor: **Caput, Daniel**
**3986 23rd Street**
**San Francisco California 94114(US)**

(74) Representative: **Goldin, Douglas Michael et al**
**J.A. KEMP & CO. 14, South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

Technical Field

This invention relates to the field of recombinant vaccines and viral diseases. More particularly, the invention relates to use of the hepatitis A virus genomic sequence to obtain oligomeric probes for diagnosis, to obtain vaccines useful in protection of subjects at risk with respect to hepatitis A, and to obtain monoclonal antibody populations useful in treatment of infected subjects and as diagnostics.

Background Art

Hepatitis is a serious debilitating disease prevalent throughout the world. It may occur in acute form and causes extensive fatigue, liver damage, and, not infrequently, is fatal. The disease is caused by a viral infection. The infective agents are endemic in underdeveloped areas of the globe, where the majority of adults carry antibodies directed against viral antigens. In the United States, Western Europe, and Japan, the incidence of hepatitis is also significant especially among those exposed to conditions which are conducive to transmission of viral infection. However, because the probability of exposure in general is lower, most adults in these areas do not carry antibodies directed against the antigens of the virus causing hepatitis, and are at high risk when travelling to areas where the virus is endemic.

Three very different and immunologically distinct forms of virus causing hepatitis are known: Hepatitis A, Hepatitis B, and a third known at present only as Non-A/Non-B. Hepatitis B virus (HBV) has been widely studied and vaccines protective against infection by HBV have been developed. Another infective species, hepatitis A virus (HAV) is a picornavirus belonging to the enterovirus genus. HAV was first characterized as the cause of acute illness by Feinstone, S. M., et al, Science (1978) 182:1026-1028; Siegl, G., et al, J Virol (1978) 26:40-47, 48-53. This form of the virus causes a clinically important disease, and more than 100,000 cases of HAV-induced hepatitis are reported annually in the United States. The incidence of the disease is similar elsewhere in the Western world.

As antibodies raised against hepatitis B are not cross-reactive against hepatitis A or protective against HAV infection, there is a need for the development of vaccines specifically directed against infection by HAV. While the virus has been characterized morphologically, and the genome partially sequenced, the development of a vaccine has been hindered by insufficient knowledge of the nucleotide sequence of the genome. As will be further explained below, vaccine design is dependent on identification of suitable epitopes either as primary peptide sequences, or resulting from production of 3-dimensional configurations, which engender the formation of neutralizing antibodies.

HAV has the typical icosahedral structure of a picornavirus, measuring 27-32 nm in diameter. The virions contain a single stranded genomic RNA molecule of approximately 7.5 kb, having positive strand polarity with a polyA stretch at the 3' end, and may be covalently linked to a protein at the 5' terminus. It has been assumed that the polypeptides encoded by the RNA are analogous to those of other picornaviruses, such as those responsible for polio and for foot and mouth disease (FMD). While variations occur, sufficient similarity has been shown between such viruses to warrant devising a systematic nomenclature of picornavirus proteins, as set forth in Rueckert, R. R., et al, Journal of Virol (1984) 50:957-959. This system of nomenclature, called the L434 convention, is based upon an idealized map of a polyprotein produced by the picornavirus genome, which can be accomodated into the pattern L-ABCD-ABC-ABCD. In this notation L is a leader protein, the first quartet represents a set of 4 capsid proteins encapsulating the viral particle, the middle triplet represents proteins of various functions, and the downstream quartet includes a proteinase capable of cleaving the polypeptide precursor of the capsid proteins into individual peptides. As shown by the invention herein, this pattern is consistent for HAV encoded polypeptides as well.

Some work relevant to vaccine preparation has been done. Monoclonal antibodies (Mabs) against HAV which are neutralizing in in vitro assays, but not protective against infection, were prepared by MacGregor, A., et al J Clin Microbiol (1983) 18:1237-1243, using spleens from mice injected with whole virus as fusion partners in preparing the Mab producing hybridomas. These Mabs appear cross-reacting, and apparently recognize antigenically similar determinants. In addition, Hughes, J. V. et al, J Virol (1984) 52:465-473 prepared Mabs which were capable of immunoprecipitating whole HAV, but not the disrupted or denatured proteins of the virus. These Mabs were obtained from hybridomas which had been formed using as fusion partners the spleens of mice which had been injected with a mixture of VP1, VP2, and VP3 HAV proteins. (Consistent with other picornaviruses, the capsid proteins appear to comprise three or four separate proteins, VP1, VP2, VP3, and possibly a VP4, of which VP1 is apparently the most exposed, and the major

provider of antigenic determinants.) It has been consistently shown that the whole virus contains antigenic sites which are not present in individual capsid proteins. (Gerlich, W. H., et al, Med Microbiol Immunol - (1983) 172:101-106; Hughes, J. V. et al (supra).)

Emini, E.A. et al, Abstracts; Modern Approaches to Vaccines (Sept 12-16. 1984, Cold Spring Harbor, NY) p72, have reported that poliovirus peptides corresponding to poliovirus capsid antigenic sites, when conjugated to BSA and injected into rabbits, rats or guinea pigs, were able to prime the subject to respond to sub-immunogenic administration of HAV by producing neutralizing antibodies.

Partial cloning and sequencing of the HAV genome has been reported by others (Ticehurst, J. R., et al, Proc Natl Acad Sci (USA) (1983) 80:5885-5889; Von der Helm, et al, J Virol Meth (1981) 3:37-44). These reports have yielded insufficient information to permit construction of polypeptides which can serve as vaccines. The present invention provides the entire genomic sequence of HAV, and thus permits manipulation of the potential epitopes to obtain effective vaccines against HAV.

EP-A-0154587, designating CH, DE, FR, GB, IT, LI and NL and published on 11 September 1985 discloses the partial sequence of the HAV genome. Certain fragments of this sequence are also disclosed.

EP-A-0175613, designating CH, DE, FR, GB, IT, LI and NL and published on 11 September 1985, discloses certain immunogenic peptides derived from HAV.

W085/01517 designating EPO contracting states AT, BG, CH, DE, FR, GB, LU, NL and SE and published on 11 April 1985 discloses a 1489 nucleotide sequence of HAV CDNA.

## Disclosure of the Invention

The invention provides a cDNA replica of the entire HAV genomic sequence. Portions of the genomic sequence are useful as probes to diagnose the presence of virus in clinical samples. The understanding of the genomic sequence also effectively makes available the entire viral polypeptide sequence and permits not only analysis, but production of portions thereof, if appropriate, in the correct configuration, so as to provide effective vaccines and permit the production of neutralizing antibodies. The availability of the entire HAV sequence thus permits the design and construction of polypeptides which may serve either as vaccines themselves, or as intermediates in the production of monoclonal antibody (Mab) preparations useful in passive immunotherapy against the disease, or as diagnostic reagents. Without the sequence of the entire genome at the disposal of the producer of the therapeutic or preventive compositions, successful production of optimally effective products would be impossible.

Accordingly, in one aspect, the invention relates to a nucleotide sequence substantially identical with that representing the entire genome of HAV as represented in Figure 1. The remaining contributions of the invention relate to utilizing this sequence or portions thereof as oligomeric probes, to produce peptides which can serve as vaccines, or as reagents to produce monoclonal antibodies useful in treatment of the disease.

Other aspects of the invention include expression systems which are capable of effecting the production of a desired protein encoded by sequences derived from the complete genome, to recombinant vectors containing such systems or portions thereof, to recombinant host cells transformed with such vectors, to proteins produced by the transformed cells, and to vaccines prepared from such proteins. In addition, the invention relates to specific peptide sequences representing epitopes encoded by the genome, and to such sequences covalently linked to carrier proteins. Such carrier proteins, in addition to more conventional carriers, include the 22 nm particle associated with hepatitis B infection, which carries polyalbumin receptor sites, and is 1000 fold more immunogenic than the unassembled subunit component. By inserting antigenic HAV determinants into the 22nm HBsAg particle, increased immunogenicity for these epitopes is obtained. The invention also relates to deleted virions capable of eliciting neutralizing antibodies in subject hosts, and to monoclonal antibodies useful in passive immunotherapy.

The invention also relates to the methods of preparing these desired polypeptide vaccines and immunoglobulins.

Insofar as the invention relates to portions of the sequence of Figure 1 and their use in expression vectors or as oligonucleotide probes, those portions of the sequences are those claimed in claim 3.

## Brief Description of the Drawings

Figure 1 shows the complete nucleotide sequence of the HAV genome. The cDNA contains the identical sequence, except, of course, that T will be substituted for U. The deduced amino acid sequence of the viral L434 polypeptide is also shown in Figure 1.

Figure 2 shows a restriction map and the regions of overlap of the four cDNA clones which comprise

the entire 7.5 kb HAV genome, pHAV47, pHAV8, pHAV1, and pHAV16.

Figure 3 shows the construction of expression vectors for sequences encoding fusion mRNAs encoding portions of superoxide dismutase (SOD) and HAV capsid protein.

Figure 4 shows a computer generated profile based on hydrophilicity and structural characteristics of the peptide chain as a function of position for regions of the polio virus polypeptide and the HAV polypeptide.

Figure 5 shows the construction of a mammalian cell expression vector encoding deleted HAV virions.

Figure 6 shows the construction of pDC-104 for production of hybrid HAV/HBsAg in yeast.

Figure 7 shows the construction of pWG-1 for production of hybrid HAV/HBsAg in yeast.

## Modes of Carrying Out the Invention

### A. Definitions

As used herein, a nucleotide sequence "substantially identical" to the exemplified HAV genome refers to a sequence which retains the essential properties of the exemplified polynucleotide. A specific, but non-limiting example of such substantial equivalence would be represented by a sequence which encodes the identical or substantially identical amino acid sequence, but, because of codon degeneracy, utilizes different specific codons. Nucleotide changes are, indeed, often desirable to create or delete restriction sites, provide processing sites, or to alter the amino acid sequence in ways which do not adversely affect functionality. "Nucleotide sequence" refers both to a ribonucleotide and a deoxyribonucleotide sequence and includes the positive sense strand, as shown, and the negative sense strand as well.

A DNA sequence "derived from" the nucleotide sequence which comprises the genome of HAV refers to a DNA sequence which is comprised of a region of the genomic nucleotide sequence, or a combination of regions of the sequence. These regions are, of course, not necessarily physically derived from the nucleotide sequence of the gene, but refer to polynucleotides generated in whatever manner which have the same or "substantially identical" sequence of bases as that in the region(s) from which the polynucleotide is derived. For example, typical DNA sequences "derived from" the HAV genome include fragments encoding specific epitopes, fragments encoding portions of the viral polypeptide, sequences encoding the capsid proteins, sequences encoding deleted virions, and sequences encoding the viral proteinase. Similarly, a peptide "derived from" the HAV polypeptide refers to an amino acid sequence substantially identical to that of this polypeptide or a portion thereof, having the same biological properties as that portion. The manner of synthesis of such "derived" peptide is not material--it may be chemical synthesis or recombinant means, for example.

"Recombinant host cells", "host cells", "cells", "cell lines", "cell cultures", and other such terms denoting microorganisms or higher eucaryotic cell lines cultured as unicellular entities, are used interchangeably, and refer to cells which can be, or have been, used as recipients for recombinant vector or other transfer DNA, and include the progeny of the original cell transfected. It is understood that the progeny of a single parental cell may not necessarily be completely identical in morphology or in genomic or total DNA complement as the original parent, due to accidental or deliberate mutation. Progeny of the parental cell which are sufficiently similar to the parent to be characterized by the relevant property, such as the presence of the nucleotide sequence encoding a desired peptide, are included in the progeny intended by this definition, and are covered by the above terms.

"Control sequence" refers to DNA sequences which are necessary to effect the expression of coding sequences to which they are ligated. The nature of such control sequences differs depending on the host organism; in procaryotes, generally such control sequences include promoter and ribosome binding site; in eucaryotes, generally, such control sequences include promoter, terminators, and, in some instances, enhancers. The term "control sequences" is intended to include, at a minimum, all components whose presence is necessary for expression, and may also include additional components whose presence is advantageous.

"Operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

### B. General Description

At the core of the present invention is the provision of a nucleotide sequence containing the entire

genome of hepatitis A virus. The availability of this complete polynucleotide, first, permits the construction of oligomers of about 20 bp or more useful in disease diagnosis. Such probes may be used to detect the presence of the viral genome in, for example, fecal filtrates of subjects suspected of harboring the virus. Knowledge of the gene sequence also enables the design and production of vaccines effective against HAV and production of neutralizing Mabs. Sequencing information available from the genome allows the amino acid sequence of the polypeptide to be deduced and compared to that of other known picornaviruses for which locations of favorable epitopes is known. Further, once the desired sequences are chosen, fragments of the genome can be obtained and expressed independently, thus providing desired polypeptides using recombinant techniques. Both procaryotic and eucaryotic hosts are useful for such expression. Short polypeptide fragments may be chemically synthesized and linked to carrier proteins for use as vaccines. Recombinantly expressed polypeptides may be provided under conditions offering a favorable environment for processing into, for example, deleted virions, thus permitting three dimensional reconstruction of epitopic sites without danger of creating an infective virus. Mammalian and yeast cells provide particularly suitable environments for such expression. In addition, the epitopes may be produced linked to a particle forming protein. The proteins thus produced may, themselves be used as vaccines, or may be used to induce immunocompetent B cells in hosts, which B cells can then be used to produce hybridomas that secrete antibodies useful in passive immunotherapy.

B.1. Preparation of the HAV Gene Sequence

The genomic sequence of HAV was prepared using stool samples of infected patients as the source for the virus. The isolated viral RNA was fractionated, and those fractions containing RNA of sufficient length to contain the entire genome were pooled, ethanol precipitated, and used to obtain a cDNA library. Colonies from the library were hybridized to oligomeric probes complementary to the 3'-proximal portions of the RNA. Several colonies hybridizing to these sequences were used to obtain additional portions of the RNA message from the cDNA library using "walking" techniques. Four cDNA clones were obtained from overlapping portions of the gene, and subjected to restriction mapping and sequencing. The entire genomic sequence was deduced from these four cDNA inserts. The amino acid sequence deduced from the viral genome is similar to that characteristic of picornaviruses, and the 5' and 3' non-coding regions probably involved in viral replication were identified.

B.2. Preparation of Viral Polypeptide Fragments in E. coli

The availability of the entire genomic sequence permits construction of expression vectors encoding presumptively antigenically active regions of the capsid proteins. Fragments encoding the desired proteins are obtained from the cDNA clones using conventional restriction digestion and ligated into vectors containing portions of fusion sequences such as $\beta$-glactosidase or SOD, preferably SOD. Any desired portion of the HAV genome could be expressed as a fusion protein or co-expressed with another readily produced protein in E. coli using this approach. However, for the purpose of generating peptides capable of raising antibodies against HAV, fragments representing portions of the genome encoding the capsid protein regions VP1, VP2 and VP3 were used. In a particularly preferred mode, human SOD encoding sequences are fused to those derived from HAV as described in more detail hereinbelow. Alternatively, since a three-dimensional antigenic site may result from suitable arrangement of individual peptide chains, bacterial expression vectors including the coding sequence for the viral proteinase, which would be encoded in the downstream quartet, may be included on the same, or cotransformed expression vector as that constructed for the precursor (PI) of the capsid proteins. Simultaneous expression of the capsid encoding and proteinase encoding sequences may also result in preparations which produce the various capsid proteins as individual peptides. (See, e.g., Klump, W., et al, Proc Natl Acad Sci (USA) (1984) 81:3351-3355; Hanecak, R., et al, Cell (1984) 37:1063-1073.) These may then assemble to the appropriate three-dimensional structure.

B.3. Preparation of Antigenic Polypeptides and Conjugation with Carrier

The availability of the entire genomic sequence also makes possible computer analysis of the structural characteristics of the deduced protein for comparison with the known sequence of polio virus. As the antigenic regions of polio virus are known, such comparisons make possible the designation of short peptide regions representing epitopes. These can then be synthesized using chemical methods, and provided with, for example, cysteine residues at the C terminus which provide means for linking the

peptides to neutral carrier proteins. A number of techniques for obtaining such linkage are known in the art, including the formation of disulfide linkages using N-succinimidyl-3-(2-pyridylthio)-propionate (SPDP) and succinimidyl 4-(N-maleimido-methyl) cyclohexane-1-carboxylate (SMCC) obtained from Pierce Company, Rockford, Illinois. These reagents create a disulfide linkage between themselves and peptide cysteine residues on one protein and an amide linkage through the ε-amino on a lysine, or other free amino group in the other. A variety of such disulfide/amide-forming agents are known. See, for example, Immun. Rev. - (1982) 62:185. Other bifunctional coupling agents form a thioether rather than a disulfide linkage. Many of these thioether-forming agents are commercially available and include reactive esters of 6-maleimidocaproic acid 2-bromoacetic acid, 2-iodoacetic acid, 4-(N-maleimido-methyl) cyclohexane-1-carboxylic acid, and the like. The carboxyl groups can be activated by combining them with succinimide or 1-hydroxy-2-nitro-4-sulfonic acid, sodium salt. The foregoing list is not meant to exhaustive, and modifications of the named compounds can clearly be used.

Any carrier may be used, which does not itself induce the production of antibodies harmful to the host, such as the various serum albumins, tetanus toxoids, or keyhole limpet hemocyanin (KLH).

The conjugates, when injected into suitable subjects, will result in the production of antisera which contain immunoglobins specifically reactive against not only the conjugates, but also against fusion proteins carrying the analogous portions of the sequence, and against appropriate determinants within whole HAV.

### B.4. Preparation of Non-Infectious (Deleted) Virions in Procaryotes or Eucaryotes

Virions which mimic the three-dimensional epitopic configuration of intact HAV may also be prepared. It is generally surmised that antigenic sites may result from three-dimensional folding of immunogenic proteins, and, specifically with respect to HAV, it has been reported that antibodies formed in response to injection with HAV capsid proteins are not reactive against the denatured viral peptides (Gerlich et al, Med Microbiol Immunol (1983) 172:101-106).

The deleted virions are prepared by providing DNA sequences which lack the 5' and 3' non-coding regions of HAV putatively responsible for replicative functions. The sequences also lack a portion of the 3' coding region presumed, by homology with other picornaviruses, to encode the replicase origin. These fragments are preferably cloned into plasmid expression vectors appropriate for yeast or mammalian cells capable of the desired post-transcription and post-translation processing; however, procaryotes can also be used. Deleted virions produced in hosts transformed with these vectors are immunoreactive against human convalescent HAV antisera, and can be used for immunization.

### B.5. Preparation of Hybrid Particle Immunogens Containing HAV Epitopes

The immunogenicity of the epitopes of HAV may also be enhanced by preparing them in mammalian or yeast systems fused with particle-forming proteins such as that associated with hepatitis B surface antigen. Constructs wherein the HAV epitope is linked directly to the particle-forming protein coding sequences produce hybrids which are immunogenic with respect to the HAV epitope. In addition, all the vectors prepared include epitopes specific to hepatitis B virus (HBV), having various degrees of immunogenicity, such as, for example, the pre-S peptide. Thus, particles constructed from particle-forming protein which include HAV sequences are immunogenic with respect to both HAV and HBV.

Hepatitis surface antigen (HBsAg) has been shown to be formed and assembled in S. cerevisiae - (Valenzuela et al, Nature (1982) 298:344-350), as well as in, for example, mammalian cells (Valenzuela, P., et al, Hepatitis B (1984), Millman, I., et al, ed, Plenum Press, pp. 225-236). The formation of such particles has been shown to enhance the immunogenicity of the monomer subunit. The constructs may also include the immunodominant epitope of HBsAg, comprising the 55 amino acids of the presurface (pre-S) region. (Neurath et al, Science (1984) 224:392-394.) Constructs of the pre-S-HBsAg particle expressible in yeast are disclosed in U.S. Serial No. 621,756, filed 18 June 1984: hybrids including heterologous viral sequences for yeast expression are disclosed in U.S. Serial No. 650,323, filed 13 September 1984. Both applications are assigned to the herein assignee and incorporated by reference. These constructs may also be expressed in mammalian cells such as Chinese hamster ovary cells using an SV40-dihydrofolate reductase vector (Michelle et al, Int Symp on Viral Hepatitis (1984)).

In addition, portions of the particle-forming protein coding sequences per se may be replaced with codons for an HAV epitope. In this replacement, regions which are not required to mediate the aggregation of units to form immunogenic particles in yeast or mammals can be deleted, thus eliminating additional hepatitis B antigenic sites from competition with the HAV epitope.

B.6. Preparation of Vaccines

Preparation of vaccines which contain peptide sequences as active ingredients is also well understood in the art. Typically, such vaccines are prepared as injectables, either as liquid solutions or suspensions; solid forms suitable for solution in, or suspension in, liquid prior to injection may also be prepared. The preparation may also be emulsified or the protein encapsulated in liposomes. The active immunogenic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the vaccine may contain minor amounts of auxiliary substances such as wetting or emulsifying agents, pH buffering agents, or adjuvants which enhance the effectiveness of the vaccine. The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkaline glycols or triglycerides; such suppositories may be formed from mixtures containing the active ingredient in the range of 0.5% to 10%, preferably 1%-2%. Oral formulations include such normally employed excipitents as, for example, pharmaceutical grades of manitol, lactose, starch magnesium stearate, sodium saccharine cellulose, magnesium carbonate and the like. These compositions take the form of solutions, suspensions, tablets, pills, capsules, sustained release formulations or powders and contain 10%-95% of active ingredient, preferably 25%-70%.

The proteins may be formulated into the vaccine as neutral or salt forms. Pharmaceutically acceptable salts, include the acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, capacity of the subject's immune system to synthesize antibodies, and the degree of protection desired. Precise amounts of active ingredient required to be administered depend on the judgment of the practititioner and are peculiar to each subject.

B.7. Preparation of Mabs Against HAV Epitopes

The immunogenic proteins prepared as described above are used to immunize mammals. Lymphocytes from these animals may be used to prepare hybridomas capable of secreting monoclonal antibodies directed against these epitopes and cross-reactive against the infective virus. The resulting monoclonal antibodies are particularly useful in diagnosis, and those which are neutralizing are useful in passive immunotherapy.

C. General Methods

The general techniques used in extracting RNA from the virus, preparing and probing a cDNA library, sequencing clones, constructing expression vectors, transforming cells, and the like are known in the art and laboratory manuals are available describing these techniques. However, as a general guide, the following sets forth some sources currently available for such procedures, and for materials useful in carrying them out.

C.1. Hosts and Expression Control Sequences

Both procaryotic and eucaryotic host cells may be used for expression of desired coding sequences when appropriate control sequences are used compatible with the designated host. Among procaryotic hosts, E. coli is most frequently used, mostly for convenience. Expression control sequences for procaryotes include promoters, optionally containing operator portions, and ribosome binding sites. Transfer vectors compatible with procaryotic hosts are commonly derived from, for example, pBR322 a plasmid containing operons conferring ampicillin and tetracycline resistance, and the various pUC vectors, which also contain sequences conferring antibiotic resistance. The foregoing operons may be used as markers to obtain successful transformants by selection. Commonly used procaryotic control sequences include the β

lactamase (penicillinase) and lactose promoter systems (Chang, et al, Nature (1977) 198:1056, the tryptophan (trp) promoter system (Goeddel, et al, Nucleic Acids Res (1980) 8:4057) and the λ derived $P_L$ promoter and N gene ribosome binding site (Schimatake, et al, Nature (1981) 292:128) and the hybrid tac promoter (De Boer, et al, Proc Natl Acad Sci (USA) (1983) 80:21-25) derived from sequences of the trp and the lac UV5 promoters. The foregoing systems are particularly compatible with E. coli; if desired other procaryotic hosts such as strains of Bacillus or Pseudomonas may be used, with corresponding control sequences.

Eucaryotic hosts include yeast and mammalian cell culture. Saccharomyces cerevisiae, or Baker's yeast and Saccharomyces carlsbergensis are the most commonly used yeast hosts, again because of convenience. Yeast compatible vectors carry markers which permit selection of successful transformants by conferring prototrophy to auxotrophic mutants or by conferring antibiotic resistance or resistance to heavy metals on wild-type strains. Yeast compatible vectors may employ the 2 micron origin of replication (Broach, J., et al, Meth Enz (1983) 101:307) the combination of CEN3 and ARS1 or other means for assuring replication, such as sequences which will result in incorporation of an appropriate fragment into the host cell genome. Control sequences for yeast vectors include promoters for the synthesis for glycolytic enzymes (Hess, et. al, J Adv Enzyme Reg (1968) 7:149, Holland, et al, Biochemistry (1978) 17:4900), and the promoter for 3 phosphoglycerate kinase (Hitzeman, et al, J Biol Chem (1980) 255:2073). For yeast expression, terminators may also be included, such as those derived from the enolase gene (Holland, M. J., J Biol Chem (1981) 256:1385). Particularly useful control systems include those specifically described herein, which comprise the glyceraldehyde-3 phosphate dehydrogenase (GAPDH) promotor or alcohol dehydrogenase (ADH) regulatable promotor, terminators also derived from GAPDH, and, if secretion is desired, leader sequence from yeast alpha factor. These systems are described in detail in U.S. Serial Nos. 468,589 and 522,909, filed 22 February 1983 and 12 August 1983, respectively, both assigned to the herein assignee, and incorporated herein by reference.

Mammalian cell lines available as hosts for expression include many immortalized cell lines available from the American Type Culture Collection, including HeLa cells, Chinese hamster ovary (CHO) cells, baby hamster kidney (BHK) cells, and a number of other cell lines. Suitable promoters for mammalian cells prominently include viral promoters such as that from Simian virus 40 (SV40) (Fiers, et al, Nature (1978) 273:113 or other viral promoters such as the Rous sarcoma virus (RSV) adenovirus, and bovine papiloma virus (BPV). Mammalian cells may also require terminator sequences. Vectors suitable for replication in mammalian cells may include viral replicons, or sequences which insure integration of the appropriate sequences into the host genome.

C.2. Transformations

The transformation procedure used depends on the host to be transformed. Bacterial transformation generally employs treatment with calcium or rubidium chloride (Cohen, S. N., Proc Natl Acad Sci (USA) (1972) 69:2110, Maniatis, et al, Molecular Cloning: A Laboratory Manual (1982) Cold Spring Harbor Press, page 254). Yeast transformations may be carried out using the method of Hinnen, et al, Proc Natl Acad Sci (1978) 75:1929-1933. Mammalian transformations are conducted using the calcium phosphate precipitation method of Graham and van der Eb, Virology (1978) 52:546, or the various modifications thereof.

C.3. Vector Construction

Vector construction employs techniques which are by now quite well understood. Site-specific DNA cleavage is performed by treating with suitable restriction enzyme under conditions which generally are specified by the manufacturer of these commercially available enzymes (see, e.g., The New England Biolabs Product Catalog). In general, about 1 µg of plasmid or DNA sequence is cleaved by 1 unit enzyme in about 20 µl buffer solution for an incubation time of about 1-2 hr at about 37° C. After incubation with the restriction enzyme, protein is removed by phenol/chloroform extraction and the DNA recovered by reprecipitation with ethanol. The cleaved fragments may be separated using polyacrylimide or agarose gel electrophoresis techniques, according to the general procedures found in Methods in Enzymology (1980) 65:499-560.

Sticky ended cleavage fragments may be blunt ended using E. coli DNA polymerase I (Klenow) in the presence of the appropriate deoxynucleotide triphosphates (dNTPs) using incubation conditions appropriate to the polymerase. The polymerase digests protruding 3' single strands, but fills in 5' protruding ends, according to the dNTPs present in the mixture. Treatment with S1 nuclease may also be used, as this results in hydrolysis of any single stranded DNA portion.

Ligations are carried out using standard buffer and temperature conditions using T4 DNA ligase, and ATP; sticky end ligations require less ATP and less ligase than blunt end ligations. When vector fragments are used as part of a ligation mixture, the vector fragment is often treated with bacterial alkaline phosphatase (BAP) in order to remove the 5' phosphate and thus prevent religation of the vector; alternatively, restriction enzyme digestion of unwanted fragments can be used to prevent religation.

Ligation mixtures are transformed into suitable cloning hosts, such as E. coli, and successful transformants selected by, for example, antibiotic resistance, and screened for the correct construction.

C.4. Construction of Desired DNA Sequences

Synthetic oligonucleotides may be prepared using an automated oligonucleotide synthesizer as described by Warner, B.D., et al, DNA (1984)3:401-411. If desired, these synthetic strands may be kinased for labeling with $^{32}P$ by using an excess of polynucleotide kinase in the presence of labeled ATP, under standard kinasing conditons.

DNA sequences including those isolated from genomic or cDNA libraries may be modified by site directed mutagenesis, as described by Zoller, M, et al, Nucleic Acids Res (1982) 10:6487-6499. Briefly, the DNA to be modified is packaged into phage as a single stranded sequence, and converted to a double stranded DNA with DNA polymerase using, as a primer, a synthetic oligonucleotide complementary to the portion of the DNA to be modified, and having the desired modification included in its own sequence. The resulting double stranded DNA is transformed into a phage supporting host bacterium, and cultures of the transformed bacteria, which will contain replications of each strand of the phage, are plated in agar to obtain plaques. Theoretically 50% of the new plaques will contain phage having as a single strand the mutated form: 50% will have the original sequence. Replicates of the plaques are hybridized to kinased synthetic probe at temperatures and conditions which permit hybridization with the correct strand, but not with the unmodified sequence. The thus identified, desired, modified sequences are then recovered and cloned to serve as sources for the desired DNA.

C.5. Hybridization with Probe

DNA libraries are probed using the procedure of Grunstein and Hogness (Proc Natl Acad Sci (USA) (1975)73:3961). Briefly, in this procedure, the DNA to be probed is immobilized on nitrocellulose filters, denatured, and prehybridized with a buffer containing 0-50% formamide, 0.6 M NaCl, 60 mM sodium citrate, 0.02% (wt/v) each of bovine serum albumin, polyvinyl pyrollidine, and Ficoll, 50 mM sodium phosphate (pH 6.5), 1% glycerine, and 100 $\mu$g/ml carrier denatured DNA. The percentage of formamide in the buffer, as well as the time and temperature conditions of the prehybridization and subsequent hybridization steps depends on the stringency desired. Oligomeric probes which require lower stringency conditions are generally used with low percentages of formamide, lower temperatures, and longer hybridization times. Probes containing more than 30 or 40 nucleotides such as those derived from cDNA or genomic sequences generally employ higher temperatures, e.g. about 40-42° and a high percentage, e.g. 50% formamide. Following prehybridization this same buffer, now containing the $^{32}P$ kinased oligonucleotide probe, is added to obtain hybridization. Radioautography of the treated filters shows the location of the hybridized probe, and the corresponding locations on replica filters which have not been probed can then be used as the source of the desired DNA.

C.6. Verification of Construction and Sequencing

For routine vector constructions, ligation mixtures are transformed into E. coli strain HB101 or other suitable host, and successful transformants selected by antibiotic resistance or other markers. Plasmids from the transformants are then prepared according to the method of Clewell, D. B., et al, Proc Natl Acad Sci (USA) (1969) 62:1159, usually following chloramphenicol amplification (Clewell, D. B., J Bacteriol (1972) 110:667). The isolated DNA is isolated and analyzed by restriction analysis, or sequenced by the dideoxy method of Sanger, F., et al, Proc Natl Acad Sci (USA) (1977) 74:5463, as further described by Messing, et al, Nucleic Acids Res (1981) 9:309, or by the method of Maxam, et al, Methods in Enzymology (1980) 65:499.

D. Examples

The following examples are intended to illustrate but not limit the invention. The procedures set forth,

for example, in ¶D.1 may, if desired, be repeated but need not be, as techniques are available for construction of the desired nucleotide sequences based on the information provided by the invention. Expression is exemplified in E. coli and in yeast, however other systems are available as set forth more fully in ¶C.1. Additional epitopes derived from the genomic structure may also be produced, and used to generate antibodies as set forth below.

D.1. Preparation of HAV cDNA

HAV virus was isolated from the stools of a hepatitis A patient collected during an epidemic outbreak in Los Angeles, California. Other isolates, available in the art could also have been used. See, for example, Daemer, R. J., et al, Infect Immunity (1981) 32:388-393; Frosner, G. G., et al, Infection, (1979) 7:303-350. Techniques for such isolation, and propagation are well known in the art.

The isolate was inoculated onto PCL/PRF/5 (Alexander hepatoma cells) followed by culture at 34°C. Detectable virus production was observed 3-4 weeks later, and the original infected cell cultures have been continuously passaged in DME/15% FCS for the past two years with no loss of virus titers. (Virus titers were measured by immunofluorescence and/or competitive radio immunoassay (HAVAB, Abbott).)

Viral RNA was extracted from the virions by conventional procedures, for example, that disclosed by Ticehurst, J. E., et al, Proc Acad Sci (USA) (1983) 80:5885-5889, including protease treatment and phenyl/chloroform extraction, followed by ethanol precipitation. Low molecular weight contaminants were fractionated from genome length RNA by 15-30% sucrose gradients run for 5 hr at 40,000 x g in a SW 41 Spinco rotor. Gradients were collected onto 22-24 fractions, each fraction precipitated, and run on a formaldehyde-agarose gel (Lehrach, H, et al, J. Biochem (1977) 16:474c. Each gel was transferred to a nitrocellulose membrane and hybridized to labeled specific probe as described by Thomas, Proc Natl Acad Sci (USA) (1980) 77:5201.

Prehybridization and hybridization were essentially as described above. Probes were kinased at the 5' terminus with T4 kinase according to the method of Lillehaug, et al, Biochemistry (1976) 15:1858 followed by purification on a Sep-pak (Millipore) filter. Two oligonucleotide probes, designated HAV 1 and 2 were constructed based on sequencing information obtained by Ticehurst, et al, (supra). The probes had the following sequences:

HAV1: 3'-ACAAATAAAGAAAATAGTCATTTA-5'

HAV2: 3'-ATGTCTGAATTTAGAATACTAACCACC-5'

These probes were thus validated by hybridization to the gels run on HAV RNA from partially purified virions.

RNA from the sucrose fractions which contain full length HAV RNA were identified by the above-described hybridization blotting techniques. Samples were pooled, ethanol precipitated and used to form a cDNA library. The cloning procedure used is a modification of that previously described by Wood and Lee, Nucleic Acid Res (1976) 3:1961; Lain et al, Cell (1979) 16:851. The two modifications introduced with respect to the published procedures are as follows: (a) a short double-stranded synthetic primer dT was annealed with the poly A+ RNA to synthesize the cDNA; (b) after dC tailing, the cDNA:RNA hybrid molecules were then annealed with PstI-cleaved dG-tailed pBR322. Before the transformation, the pBR322:mRNA:cDNA molecules were repaired with concentrated extract of activated Xenopus eggs which was prepared as indicated below. The ligation mixtures were than transformed into E. coli K12 MC1061 to $Amp^R$.

Xenopus laevis eggs were obtained as described by Newport and Kirschner, Cell (1982) 30:675-686, and were washed twice with MMR (0.1 M NaCl, 2 mM KCl, 1 mM $MgSO_4$, 2 mM $CaCl_2$, 5 mM Hepes, pH 7.8, 0.1 mM EDTA) and dejellied in 2% cysteine. The eggs were then washed twice with MMR/4 and activated by electric shock (5 sec, 12 V); incubated 3 hours at room temperature, followed by 2 washed with 160 mM $\beta$-glycerophosphate, 2 mM EGTA, 10 mM $MgCl_2$, 10% glycerol, 1 mM PMSF, 25 $\mu$g/ml creatine-phosphokinase, 5 mM phosphocreatine, pH 7.5; and then crushed by pipetting and centrifuged for 10 minutes at 10,000 rpm. The crude extract was collected through the lipid layer and recentrifuged for 30 minutes at 40,000 rpm. The clear extract was aliquoted and stored frozen at -80°C.

The resulting cDNA library was probed by transferring the colonies to replicate nitrocellulose filters, and hybridizing to HAV1 and HAV2 as described above. Using the foregoing probes, and selecting colonies hybridizing to both, 3' proximal cloners were obtained. Further portions of the genome were identified by "walking" techniques. An additional clone was obtained using the 5' terminal PstI/PstI fragment from pHAV1307 as probe (Ticehurst, J., et al, (Supra)).

Four recombinant cDNA inserts designated pHAV16, pHAV1, pHAV8, and pHAV47 were selected and restriction maps prepared. The overlapping regions were confirmed by cross-hybridization studies, and the

total length of non-overlapping regions was approximately 7.5 kb, the entire span of the HAV genome. The results of the restriction mapping and overlap are shown in Figure 2. pHAV16, pHAV1, pHAV8 and pHAV47 were deposited with the American Type Culture Collection (ATCC) on 29 March 1985 and assigned accession numbers 53077, 53078, 53076 and 53079, respectively.

Complete sequencing of the series of four inserts shown in Figure 2 gave the results for the corresponding viral RNA set forth in Figure 1. (Analysis of the open reading frames associated with this sequence indicated a deletion of 22 nucleotides at position 3530 of the HAV genome; this was corrected by site-directed metagenesis to obtain the sequence shown in Figure 1; the corrected plasmid was designated pHAV1*.)

The 7,478 nucleotide heteropolymeric sequence is followed by a polyA tract of undetermined length. A single open reading frame starts with an AUG triplet at nucleotide 734 and terminates with a UGA codon at nucleotide 7,415. Thus, the polyprotein encoded is 2227 amino acids and has a molecular weight of 251,940 daltons. The deduced genomic RNA sequence in Figure 1 shows the 5' non-coding region required for linkage to a tyrosine residue on a viral protein, and the first 75 nucleotides of the sequence can be arranged in the highly stable secondary structure comprising stems and loops known from other picornavirus RNA sequences. Also, the 5' terminal portion contains long U + C repeats, as is generally observed in other picornaviruses.

D.2. Construction of Expression Vectors and Expression of HAV Sequences in E. coli

Four bacterial expression plasmids were constructed which directed the simultaneous synthesis of human superoxide dismutase (SOD) (Hallewell, et al, Nucleic Acids Res (1985) 13:2017 and also portions of all of the hepatitis A capsid precursor protein, P1 (Najarian, et al, Proc Natl Acad Sci (USA) (1985) 82:2627. Three of the plasmids synthesized part or all of the P1 fused to the carboxy terminus of SOD. The fourth plasmid also generated a fused mRNA for SOD and P1 gene sequences but contained a sequence between the SOD and P1 genes which provided a stop codon for translation termination of SOD along with a ribosome binding site for reinitiation of translation at the methionine codon thought to be the natural initiator codon of P1. The constructions are shown schematically in Figure 3.

The expression plasmids were based on the tac promoter driven expression plasmid pSOD16 of Hallewell, et al, (supra). Plasmid pSOD16cf2 was generated from pSOD16 by replacement of a portion of the carboxy terminal coding region of the SOD gene and downstream polylinker sequence through the EcoRI site by the new polylinker sequence

```
5' GATCGCCATGGGTACCCGGGTCGACTAAATGACTAG 3'
     3' CGGTACCCATGGGCCCAGCTGATTTACTGATCTTAA 5'
```

The substitution of this polylinker sequence results in the removal of the natural carboxy terminal Gln of SOD.

pSODHAV2-2

A 1390 base pair BglII(repair)/XmnI fragment isolated from a digest of pHAV8116SV7d (see ¶ D.4) was cloned into the SmaI site of pSOD16cf2. DNA of a resulting clone having the HAV insert in the proper orientation was digested with KpnI, the 3' overhang was removed with T4 DNA polymerase, then digested the HindIII, and this site was filled in with reverse transcriptase. The resultant linear, blunt ended DNA was religated and transformed into E. coli strain D1210 (Salder, et al, Gene (1980) 8:279-300) to obtain pSODHAV2-2.

pSODHAV2-2 was transformed into E. coli RRIΔM15 (Ruther, U., Nucleic Acids Res (1982) 10:5765-5772) for expression to obtain a fusion protein of SOD with the natural SOD carboxy terminal Gln replaced by Met-Glu followed by HAV sequence beginning with the Leu encoded at HAV nucleotide 2072 of the VP3 capsid protein and extending through VP1 to the Val encoded at nucleotide 3201 and terminated with the sequence Gly-Arg-Leu-Asn- Asp encoded by the polylinker.

pSODHAVP1/1

pSODHAVP1/1 contains, in addition to the sequences in pSODHAV2-2, the remainder of VP3 and the

carboxy half of VP2. pSODHAV2-2 was digested with NcoI (5' to the HAV sequences) and with SacI, which cuts within the VP1 sequence. The 3.4 kb fragment containing all the SOD sequences and a part of the VP1 sequences was gel isolated, ligated to a 1806 bp NcoI/SacI fragment isolated from pHAV81167d (see ¶ D.4) and transformed into E. coli strain D1210 to obtain pSODHAVP1/1.

When expressed in E. coli strain RRIΔM15, SODHAVP1/1 generated a fusion protein of SOD with HAV sequences starting at the Val of VP2 encoded at nucleotide 1182 through Val of VP1 encoded at nucleotide 3201.

pSODHAV9-1

pSODHAV9-1 was derived from pSODHAVP1/1 and contains the additional half of VP2 to generate the full P1 precursor sequence. A 438 bp BstXI fragment from PHAV47 (see ¶ D.1) containing the 5' end of VP2 was gel isolated and ligated to the single stranded synthetic DNA oligomer,

5'-ATGAATATGTCCAAACAAGGAATTTTCCAGACTG-3'

via the BstXI overhang at the 3' end of the 438 bp fragment. The single stranded sequence corresponds to the coding strand of the HAV genome from the extreme 5' end of the P1 precursor. The oligomer was phosphorylated at its 5' end and the strand complementary to the oligomer was synthesized with reverse transcriptase using the 3' hydroxyl of the BstXI overhang of the 438 bp fragment as primer. The resulting fragment was then digested with NcoI, and the NcoI/blunt fragment including the added 30 base pairs of double stranded oligomer, was gel isolated and ligated to pSODHAVP1/1, which had been previously digested with NcoI, at room temperature for 2 hr. The reaction was diluted, and the four dNTPs, Klenow, and additional ligase added to circularize the DNA. The ligation mixture was transformed into E. coli RRIΔM15 to obtain pSODHAV9-1 which contains HAV nucleotides 734 to 3201 (VP2, VP3, VP1).

pSODHAV10-5

pSODHAV10-5 was constructed in a manner analogous to that described above for pSODHAV9-1 except that the synthetic DNA oligomer

5' GATAGAGGAATTATAAGATGAATATGTCCAAACAAGGAATTTTCCAGACTG 3'

was used. The oligomer provides for stop codon and a ribosome binding site, in addition to HAV sequences, downstream. pSODHAV10-5 when expressed results in free SOD with the natural terminal Gln replaced with the sequence Met-Ile-Glu-Glu-Leu, and also free HAV P1 lacking its 13 carboxy terminal amino acids, which were replaced with Gly-Arg-Leu-Asn-Asp.

Expression

E. coli strain RRIΔM15, Ruther, U. (supra), cells were transformed with the plasmids described above and single colony transformants were grown overnight at 37°C in 2 ml L-broth plus 100 μg/ml ampicillin. Glyercol (50%) stocks of these cultures were prepared and stored at -20°C.

For protein expression analysis, overnight cultures, in medium as above, were begun from glycerol stocks. These cultures were diluted 1/100 into the same medium and grown at 37°C to an $OD_{650}$ of 0.5-0.8. Uninduced controls were prepared by diluting a 1 ml portion of the growing culture with an equal volume of the same medium. Tac promoter-driven protein synthesis was induced either by diluting of 1 ml of growing culture with medium plus 2 mM IPTG (Isopropyl β-thiogalactopyranoside) or by addition of 1/100 volume of 100 mM IPTG to larger volume cultures, in either case, to give 1 mM IPTG, for 3-4 hr at 37°C. Large scale cultures had 50 ml medium in 500 ml flasks or 250 ml medium in liter flasks.

Cells were lysed in the presence of SDS and DTT for analysis on denaturing polacrylamide gels (Leammli, Nature (1970) 277:680) or with glass beads for analysis on non-denaturing polyacrylamide gels.

Expression was detected by the appearance of (a) protein band(s) of expected size present in induced sample lysates but not in uninduced samples; induced proteins were detected at their respective, expected molecular weights for all plasmids.

The HAV component of fusions and the free P1 of pSODHAV10-5 were detected by Coomassie staining and by specific reaction with anti-HAV antibodies. The SOD component of fusions was detected by Coomassie staining and by anti-SOD antibodies; free SOD was detected both by Coomassie stain and by

enzymatic activity in native gels. Gels were either stained with Coomassie blue or subjected to Western analysis after electroblotting onto nitrocellulose filters (Towbin et al, Proc Natl Acad Sci (USA) (1979) 76:3450); blots were preincubated with 0.3% Tween 20 in PBS for 1 hr at room temperature or overnight at 4°C, treated with anti-HAV or anti SOD at 1:100 dilution, and incubated for 1 hr at room temperature. (Rabbit anti-HAV was prepared using partially disrupted HAV virions. Anti-human SOD was prepared by sequential exposure of rabbits to purified recombinant human SOD produced in yeast (see EPO 138 111).) Blots were washed extensively in 0.3% Tween/PBS and incubated for 1 hr at room temperature with a 1:200 dilution of horseradish peroxidase conjugated goat anti-rabbit-IgG antibody, then washed and treated with Bio-Rad HRP color reagent at 0.5 mg/ml in 8 mM Tris HCl pH 7.5, 16 mM NaCl, 0.015% $H_2O_2$, 16% methanol.

SOD activity in non-denaturing gels was identified by the method of Beauchamp, C., et al, Anal Biochem (1971) 44:276. Briefly, gels were soaked in the absence of light, first in 2.5 mM nitro blue tetrazolium for 20 min at room temperature, then in 36 mM $KPO_4$, pH 7.8, 29 mM TEMED, 28 $\mu$M riboflavin for 15 min at room temperature. Buffer was removed and the gel was illuminated for 15 min from below using a fluorescent light box. The results of these analyses are shown in Table 1.

Table 1

| Plasmid | MW (induced) Calculated | Observed | Coomassie staining (PAGE) | Western with anti-HAV antibody | SOD activity |
|---|---|---|---|---|---|
| pSODJAV2-2 | 59,120 | 58,880 | + | + | nt |
| pSODHAVP1/1 | 92,160 | 88,820 | + | + | – |
| pSODHAV9-1 | 108,910 | 105,900 | – | + | nt |
| pSODHAV10-5 | 92,970(P1) | 84,140 | – | + | – |
| | 16,440(SOD) | 20,650 | + | – | + |

nt=not tested

Expressed products were approximately 1-3% of total cell protein for pSODHAV2-2 and pSODHAV1/1 and about 50-fold lower for pSODHAV9-1 and pSODHAV10-5.

The SODHAV2-2 and SODHAVP1/1 fusion proteins were purifed on preparatory SDS-acrylamide gels, located by Coomassie staining, and electroeluted. These purified proteins were injected into mice and the resulting sera were tested for reactivity with HAV virus on Western blots and in ELISA assays. Mouse antisera against the SODHAV2-2 react with a 34 kd protein from HAV virus on Western blocks that appears to be HAV-VP1 and was therefore also designated anti-SOD-VP1. In a capture ELISA both mouse anti-SODHAV2-2 and anti-SODHAV1/1 react with HAV virus.

In the ELISA assay, microtiter wells were coated overnight with IgG from an HAV seropositive patient. The coated wells were washed and incubated 1 hr with purified HAV. Dilutions of the mouse sera were the incubated in the well for 1 hr. The wells were then incuated 1 hr with peroxidase conjugated goat anti-mouse Ig followed by reaction with the peroxidase substrate 2,2'azino-di-[3-ethylbenzthiazoline sulfonate] (ABTS) and $H_2O_2$, and the plates were read on an ELISA reader at 414 nm; the titer is expressed as the reciprocal of the dilution that gives 50% of the maximum absorbance. The results are as follows:

| | |
|---|---|
| Control Mouse a | < 10 |
| Control Mouse b | < 10 |
| Mouse Anti-SOD-VP1 a | 308 |
| Mouse Anti-SOD-VP1 b | 158 |
| Mouse Anti-SOD-P1 a | 183 |
| Mouse Anti-SOD-P1 b | 160 |
| Mouse Anti-SOD-P1 c | 30 |
| Mouse Anti-SOD-P1 d | 42 |

In additional assays, 20 ng of SODHAV2-2 and SODHAVP1/1 were spotted onto nitrocellulose strips, and the strips were then reacted with two HAV seropositive human sera and two seronegative human sera. The strips were washed and reacted with peroxidase conjugated goat anti-human IgG, and then reacted with the peroxidase substrate 4-chloro-1-naphthol to visualize the binding of the peroxidase conjugated antibody. The results showed that the SODHAVP1/1 reacted with both the seropositive samples and neither of the seronegative samples; the SODHAV2-2 reacted with one of two seropositive samples and neither seronegative sample.

In another assay, SODHAVP1/1 was used as the coating antigen in an ELISA assay with HAV seronegative and HAV seropositive human and chimpanzee sera. All four positive sera showed a greater reaction with the SODHAVP1/1 than did the four negative sera. In this assay, microtiter wells were coated with solution of 5 µg/ml of SODHAVP1/1. The coated wells were then reacted with a 1:50 dilution of the various chimp and human sera for 1 hr, and were then incubated with peroxidase conjugated goat anti-human IgG (which cross-reacts with chimp IgG). The wells were finally incubated with ABTS and $H_2O_2$, and read on an ELISA reader at 414 nm. The results are given in absorbance units.

| Serum | HAV Reactive | |
|---|---|---|
| Human-1 | - | 0.211 |
| Human-2 | - | 0.346 |
| Chimp-1 | - | 0.459 |
| Chimp-2 | - | 0.100 |
| Human-3 | + | 0.787 |
| Human-4 | + | 0.483 |
| Human-5 | + | 0.788 |
| Chimp-3 | + | 1.224 |

D.3. Construction of Amino Acid Sequences Corresponding to Putative Epitopes

Three short amino acid sequences were synthesized based on analysis of the deduced amino acid sequence of HAV and comparison with the known epitopes of polio virus protein. The hydrophilicity and structural characteristics as a function of position for both the polio polypeptide and the HAV polypeptide were compared with the aid of a computer-based analysis. "Peaks" representing putative areas of structural homology have been identified with corresponding Roman numerals and are shown in Figure 4.

Areas III and V of the polio virus polypeptide represent major antigenic sites of VP1 (Minor et al, Nature (1983) 301:674-679; Evans et al, Nature (1983) 304:459-462). Based on this information, and upon the homology between HAV and polio, two polypeptides comprising sequences that give rise to the corresponding HAV peaks Va and IIIa (Figure 4) were synthesized. A third polypeptide corresponding to an HAV region with characteristics similar to those of Va and IIIa but with no homologous region in the polio sequence was also synthesized.

The three sequences constructed are as follows, where the numbers correspond to the positions designated in the deduced sequence of Figure 1.

Peak IIIa:

```
      561           565
      Pro-Glu-Leu-Lys-Pro-Gly-Glu-Ser-Arg-
570              575 576
His-Thr- Ser-Asp-His-Met-Ser-Cys;
```

Peak Va

```
      591           595
      Thr-Phe-Asn-Ser-Asn-Asn-Lys-Glu-Tyr-
600    602
Thr-Phe-Pro-Cys;
```

No Polio Homology

```
   606           610           615
   Ser-Ser-Thr-Ser-Asn-Pro-Pro-His-Gly-Leu-Pro-
 618
Ser-Thr-Cys.
```

Each of these sequences ends with a C-terminal cysteine for use in conjugation by a covalent bond to a carrier protein of sufficient size to confer immunogenicity of the peptide epitope.

The synthesized proteins were conjugated to the carrier protein, keyhole limpet hemocyanin (KLH) by disulfide linkage through the cysteine of the peptide using m-maleimido-benzoyl-N-hydroxysuccinimide ester (MBS) as the coupling reagent (Liu et al, Biochem (1979) 18:690-697; Green, N., et al, Cell (1982) 28:477-487). Alternatively, the peptides were coupled to KLH using glutaraldehyde (0.05-0.3%).

The conjugated peptides were injected into rabbits. Sera obtained after 31-100 days contained antibodies against the injected conjugates as assayed by ELISA. Western blots using the antisera against gels run on tissue culture-produced HAV show immunoprecipitates for the antisera produced in response to the protein corresponding to peak Va and to the protein with no polio homology. Immunoprecipitation was observed with the bands corresponding to VP1 protein.

D.4. Preparation of Non-Infectious (Deleted) Virions

Deleted virions can be prepared using expression vectors compatible with procaryotic, yeast, and mammalian cells.

The P1 precursor of several picornaviruses synthesized in vitro can be processed into individual virion proteins which then assemble into immunoreactive virion-like particles (Nicklin, et al, Biotechnology (1986) 4:33; Palmenberg, et al, J. Virol (1979) 32:770; Shih, et al, Proc Natl Acad Sci (USA) (1978) 75:5807; Hanecak, et al, Proc Natl Acad Sci (USA) (1982) 79:3973; Grubman, et al, J Virol (1985) 56:120).

To obtain a similar effect in vivo, the E. coli transfected with pSODHAV10-5, which produces the P1 precursor (see ¶D.2) can be modified to produce the virus encoded proteinase (3C) along with the P1 protein. The resulting combination of P1 precursor coexpressed with proteinase will yield non-infectious, assembled virion-like particles.

Similar constructs expressing the P1 precursor and proteinase can be produced in yeast and

mammalian cells. For production of non-infectious virion-like particles, mammalian cells can be transfected with the HAV genome under the control of appropriate regulatory elements. By deleting the 3' end portion of the genome beyond the sequences encoding 3C (ie., the 3D region) replicase gene is removed, thereby crippling the replication ability of the virus. The transfected cells will produce all virion proteins from the P1 and P2 region in addition to 3A, 3B and 3C. This is sufficient to generate virion-like particles, but will not generate infectious virus.

For example, an expression vector compatible with mammalian cells pHAV8161-SV7d, is constructed as shown in Figure 5. The HAV coding sequences are provided by digestion of pHAV1*, pHAV8, and pHAV16 (¶D.1). The mammalian replication and control systems are provided by pSV7d (see below), which is a pBR322 derivative modified to contain the SV40 origin of replication, early promoter and polyadenylation site. It also has a polylinker with several restriction sites downstream from the SV40 promoter, as well as TAA stop codons in all three reading frames downstream from the polylinker.

In more detail, pSV7d was constructed as follows: the 400 bp BamHI/HindIII fragment containing the SV40 origin of replication and early promoter was excised from pSVgtI (Mulligan et al, Mol Cell Biol (1981) 1:854-864) and purified. The 240 bp SV40 BclI/BamHI fragment containing SV40 polyA addition sites was excised from pSV2/dhfr (Subramani et al, J Mol Cell Biol (1981) 1:854-864) and purified. The fragments were fused through the linker:

```
                     Stop Codons
                      1   2   3

5'-AGCTAGATCTCCCGGGTCTAGATAAGTAAT-3'
         TCTAGAGGGCCCAGATCTATTCATTACTAG

HindIII  BglII SmaI  XbaI       BclI overhang
```

which contains the restriction sites shown and stop codons in all three reading frames. The resulting 670 bp. fragment, containing SV40 origin of replication, SV40 early promoter, polylinker with stop codons and SV40 polyadenylation site was closed into the BamHI site of pML, a pBR322 derivative with an about 2.5 kb deletion (Lusky and Botchan, Cell (1984) 36:391), to yield pSV6. The EcoRI and EcoRV sites in the pML sequences of pSV6 were eliminated by digestion with EcoRI and EcoRV, treatment with Bal31 nuclease to remove about 200 bp per end, and religation to yield pSV7a. (The Bal31 resection also eliminated one BamHI restriction site flanking the SV40 region approximately 200 bp away from the EcoRV site. To eliminate the other BamHI site flanking the SV40 region, pSV7a was digested with NruI, which cuts in the pML sequence upstream from the origin of replication, and recircularized by blunt end ligation to yield pSV7b.

pSV7c and pSV7d represent successive polylinker replacements. First, pSV7b was digested with StuI and XbaI, and the linker:

```
        BglII EcoRI  SmaI   KpnI XbaI

   5'-AGATCTCGAATTCCCCGGGGGTACCT
       TCTAGAGCTTAAGGGGCCCCCATGGAGATC
```

was ligated into the vector to yield pSV7c.

pSV7c was digested with BglII and XbaI, and the linker:

```
  BglII EcoRI   SmaI XbaI  BamHI SalI

 5'-GATCTCGAATTCCCCGGGTCTAGAGGATCCGTCGAC
         AGCTTAAGGGGCCCAGATCTCCTAGGCACGTGATC
```

was ligated to yield pSV7d.

In the construction of pHAV8161-SV7d, pHAV161 (supra) was digested with BamHI and SalI to release the 4.2 kb fragment encoding a portion of the HAV sequence. pHAV8 was digested with SalI and BamHI to provide the coding sequences upstream of the SalI site in the pHAV161 insert. These two fragments were ligated into the BamHI site of pSV7d. The resulting vector pHAV8161-SV7d places the ligated HAV sequences encoding amino acid 1-2076 under control of the SV40 promotor and directly behind the vector provided stop codon, as shown in Figure 8.

pHAV8161-SV7d may be transfected into COS cells and transient expression tested by immunofluorescence using human convalescent serum for HAV. Plasmid DNA from successful COS transformants may then be used to construct vectors for stable expression.

D.5. Hybrid Particle HAV Immunogens

U.S. Serial No. 650,323, filed 12 April 1984 and assigned to the same assignee is incorporated herein by reference. This application describes the construction of hybrid particles of hepatitis B surface antigen (HBsAg) containing inserts of foreign immunogens into a pre-surface (pre-S) coding portion in reading frame with the codons for HBsAg. Plasmid pDC101, described therein contains a portion of the pre-S/HBsAg gene, including 55 codons of the pre-S region, in a GAPDH controlled expression cassette cloned into the BamHI site of pBR322 derivative. The incorporated application describes the insertion of desired immunogens, such as the gD (glycoprotein D) antigenic site into a unique EcoRI site present in the pre-S region of pDC101 to give the hybrid plasmid pDC103. Similarly, in accordance with the present invention, desired epitopes derived from the HAV genome may be provided with suitable EcoRI linkers and inserted in proper reading frames into the EcoRI site of pDC101, or used to replace the gD codons in the pDC103 hybrid. pDC103 is deposited with ATCC and has accession no. 20726.

The construction of pDC-104, a yeast expression vector for a hybrid gene containing the sequence encoding HAV-VP1 epitope shown in Figure 6 was as follows. The HindIII/SalI fragment obtained from HAV1307 (and containing codons for amino acids 445-657 of the VP1 region) was provided with synthetic EcoRI linkers and ligated into the EcoRI site of pDC103 in place of the gD sequences. The resultant was transformed into yeast host *S. carlsbergensis* 2150-2-3 and the cultured hosts harvested. The hybrid HBV/HAV particle is detected in lysates of the yeast cells by using immobilized HBV antisera to capture the antigen and a second soluble antiserum to HAV labeled with horseradish peroxidase. Alternatively, bound anti-HAV antisera can be used to capture the particle, followed by a second labeled anti-HBV antiserum.

pWG-1, a yeast expression vector containing the coding sequence for the hybrid gene harboring the VP-1 neutralizing epitope in the particle-forming portion of the HBsAg-encoding gene is constructed similarly (see Figure 7). The DNA corresponding to the HAV epitope of the following sequence:

```
  1   GATCCAGTTTTAGCAAAGAAGGTACCTGAGACATTTCCTGAATTGAAGCCTGGAGAGTCC
          GTCAAAATCGTTTCTTCCATGGACTCTGTAAAGGACTTAACTTCGGACCTCTCAGG

 61   AGACATACATCAGATCACATGTCTATTTATAAATTCATGGGAAGGTCTCATTTTTTGTGC
      TCTGTATGTAGTCTAGTGTACAGATAAATATTTAAGTACCCTTCCAGAGTAAAAAACACG

121   ACTTTTACTTTCAATTCAAATAATAAAGAGTACACATTTCCAATCG
      TGAAAATGAAAGTTAAGTTTATTATTTCTCATGTGTAAAGGTTAGCCTAG
```

codes for amino acids 792-848 and is synthesized using a commercially available automated oligonucleotide synthesizer. The resulting synthetic oligomer, containing BamHI sites, is then cloned into the BglII sites of the host vector pDC101, described in U.S. 650,323 (supra), replacing a 150 bp fragment of the HBsAg coding for a dominant epitope. The resulting vector pWG-1 is transformed into *S. carlsbergensis* as above, and the transformants selected for $LEU^+$. Successful transformants are cultured and grown, and the modified HBsAg particles recovered from the medium, and their bifunctional character confirmed as above.

Hybrid particle immunogens are thus prepared using fused coding sequences for HBsAg and HAV and provide enhanced immunogenicity for the HAV epitopes.

**Claims**

**Claims for the following Contracting States : CH, DE, FR, GB, LI, NL**

**1.** A nucleotide sequence substantially identical with that of the HAV genome as represented in Figure 1.

**2.** A nucleotide sequence encoding a viral polypeptide substantially identical with that encoded by the HAV genomic sequence shown in Figure 1.

**3.** A recombinant expression system comprising a coding portion derived from the sequence of Claim 1 operably linked to a control sequence compatible with a desired host; the coding portion being any portion other than fragments substantially identical to nucleotides 328-3305, 2074-3047, 1812-3305, 1969-3047, 1806-3305, 1808-1834, 2207-2239, 2288-2323, 2324-2413, 2474-2530 2675-2716 or 23-3295 of the sequence represented in Figure 1.

**4.** Recombinant host cells transformed with the expression system of Claim 3.

**5.** Protein produced by the cells of Claim 4.

**6.** The system of Claim 3 which further includes, contiguous to said coding portion, and in reading frame therewith, a fused nucleotide sequence encoding a non-HAV encoded protein or portion thereof.

**7.** A recombinant expression system comprising a coding portion derived from the sequence of Claim 1 operably linked to a control sequence compatible with a desired host which further includes, upstream of said coding portion, and separated by a stop codon, a fused nucleotide sequence encoding a non-HAV encoded protein or portion thereof.

**8.** The system of claim 6 or 7 wherein the fused DNA sequence encodes human superoxide dismutase or portion thereof.

**9.** The system of claims 6 or 7 wherein the fused DNA sequence encodes a non-HAV particle forming protein.

**10.** Recombinant host cells transformed with the system of Claim 6 or 7.

**11.** Protein produced by the cells of Claim 10.

**12.** The system of Claim 3 wherein the coding portion is the nucleotide sequence of Claim 1 between nucleotide positions selected from the group consisting of 2072-3201; 1182-3201; and 734-3201.

**13.** An antigenic peptide comprising a sequence derived from the sequence of claim 1 covalently linked at the C terminus to cysteine which is selected from the group:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; and

Ser-Ser-Thr-Ser-Asn-Pro-Pro-His-Gly-Leu-Pro-Ser-Thr-Cys.

**14.** The protein according to claim 5, wherein said protein contains an epitope of HAV coupled to a non HAV particle forming protein to form a complex, such that the complex is capable of forming a particle immunogenic against HAV infection.

**15.** The protein according to Claim 14 wherein the epitope is derived from amino acids 445-657 or 792-848 of the HAV polypeptide sequence.

**16.** An oligonucleotide useful for detecting the presence of HAV in a biological sample which comprises a DNA sequence derived from the nucleotide sequence in Figure 1; the oligonucleotide being a fragment according to claim 3.

**17.** A protein according to any one of Claims 5, 11 and 13-15 for use in a vaccine effective against Hepatitis A virus.

**18.** An expression system for a deleted HAV virion which comprises a nucleotide sequence encoding the P1 precursor protein as shown in Figure 1, said nucleotide sequence operably linked to suitable control sequences, and transformed into a host cell capable of producing a protease effective in cleaving said P1 precursor to obtain a deleted HAV virion.

**19.** The expression system of claim 18 wherein said protease is encoded by the 3C HAV region of Figure 1.

**20.** An expression system according to claim 18 wherein said deleted virion is encoded by a DNA corresponding to nucleotides 734-5767 of the sequence represented in Figure 1.

**21.** Protein produced by an expression system according to any one of claims 18 to 20.

**22.** An expression system according to claim 3 wherein said nucleotide sequence encodes VP2.

**23.** A process for obtaining protein comprising HAV sequences which comprises culturing cells according to claim 4 or claim 10, or cells containing an expression system according to claim 18, and recovering the protein thus obtained.

**Claims for the following Contracting States : AT, BE, LU, SE**

**1.** A nucleotide sequence substantially identical with that of the HAV genome as represented in Figure 1.

**2.** A nucleotide sequence encoding a viral polypeptide substantially identical with that encoded by the HAV genomic sequence shown in Figure 1.

**3.** A recombinant expression system comprising a coding portion derived from the sequence of Claim 1 operably linked to a control sequence compatible with a desired host; the coding portion being any portion other than fragments substantially identical to nucleotides 23-3295 of the sequence represented in Figure 1.

**4.** Recombinant host cells transformed with the expression system of Claim 3.

**5.** Protein produced by the cells of Claim 4.

**6.** The system of Claim 3 which further includes, contiguous to said coding portion, and in reading frame therewith, a fused nucleotide sequence encoding a non-HAV encoded protein or portion thereof.

**7.** The system of Claim 3 which further includes, upstream of said coding portion, and separated by a stop codon, a fused nucleotide sequence encoding a non-HAV encoded protein or portion thereof.

**8.** The system of Claim 6 or 7 wherein the fused DNA sequence encodes human superoxide dismutase or portion thereof.

**9.** The system of claims 6 or 7 wherein the fused DNA sequence encodes a non-HAV particle forming protein.

**10.** Recombinant host cells transformed with the system of Claim 6 or 7.

**11.** Protein produced by the cells of Claim 10.

12. The system of Claim 3 wherein the coding portion is the nucleotide sequence of Claim 1 between nucleotide positions selected from the group consisting of 2072-3201; 1182-3201; and 734-3201.

13. An antigenic peptide comprising a sequence derived from the sequence of claim 1 covalently linked at the C terminus to cysteine which is selected from the group:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; and

Ser-Ser-Thr-Ser-Asn-Pro-Pro-His-Gly-Leu-Pro-Ser-Thr-Cys.

14. The protein according to claim 5, wherein said protein contains an epitope of HAV coupled to a non HAV particle forming protein to form a complex, such that the complex is capable of forming a particle immunogenic against HAV infection.

15. The protein according to Claim 14 wherein the epitope is derived from amino acids 445-657 or 792-848 of the HAV polypeptide sequence.

16. An oligonucleotide useful for detecting the presence of HAV in a biological sample which comprises a DNA sequence derived from the nucleotide sequence in Figure 1; the oligonucleotide being other than the fragment substantially identical to nucleotides 23-3295 of the sequence represented in Figure 1.

17. A protein according to any one of Claims 5, 11 and 13-15 for use in a vaccine effective against Hepatitis A virus.

18. An expression system for a deleted HAV virion which comprises a nucleotide sequence encoding the P1 precursor protein as shown in Figure 1, said nucleotide sequence operably linked to suitable control sequences, and transformed into a host cell capable of producing a protease effective in cleaving said P1 precursor to obtain a deleted HAV virion.

19. The expression system of claim 18 wherein said protease is encoded by the 3C HAV region of Figure 1.

20. An expression system according to claim 18 wherein said deleted virion is encoded by a DNA corresponding to nucleotides 734-5767 of the sequence represented in Figure 1.

21. Protein produced by an expression system according to any one of claims 18 to 20.

22. An expression system which comprises a nucleotide sequence encoding a protein selected from the group consisting of VP1, VP2 and VP3 operably linked to suitable control sequences.

23. A process for obtaining protein comprising HAV sequences which comprises culturing cells according to claim 4 or claim 10, or cells containing an expression system according to claim 18, and recovering the protein thus obtained.

**Claims for the following Contracting State : IT**

1. A nucleotide sequence substantially identical with that of the HAV genome as represented in Figure 1.

2. A nucleotide sequence encoding a viral polypeptide substantially identical with that encoded by the HAV genomic sequence shown in Figure 1.

3. A recombinant expression system comprising a coding portion derived from the sequence of Claim 1 operably linked to a control sequence compatible with a desired host; the coding portion being any portion other than fragments substantially identical to nucleotides 328-3305, 2074-3047, 1812-3305, 1969-3047, 1806-3305, 1808-1834, 2207-2239, 2288-2323, 2324-2413, 2474-2530 or 2675-2716 of the sequence represented in Figure 1.

4. Recombinant host cells transformed with the expression system of Claim 3.

5. Protein produced by the cells of Claim 4.

6. The system of Claim 3 which further includes, contiguous to said coding portion, and in reading frame therewith, a fused nucleotide sequence encoding a non-HAV encoded protein or portion thereof.

7. A recombinant expression system comprising a coding portion derived from the sequence of Claim 1 operably linked to a control sequence compatible with a desired host which further includes, upstream of said coding portion, and separated by a stop codon, a fused nucleotide sequence encoding a non-HAV encoded protein or portion thereof.

8. The system of claim 6 or 7 wherein the fused DNA sequence encodes human superoxide dismutase or portion thereof.

9. The system of claims 6 or 7 wherein the fused DNA sequence encodes a non-HAV particle forming protein.

10. Recombinant host cells transformed with the system of Claim 6 or 7.

11. Protein produced by the cells of Claim 10.

12. The system of Claim 3 wherein the coding portion is the nucleotide sequence of Claim 1 between nucleotide positions selected from the group consisting of 2072-3201; 1182-3201; and 734-3201.

13. An antigenic peptide comprising a sequence derived from the sequence of claim 1 covalently linked at the C terminus to cysteine which is selected from the group:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; and

Ser-Ser-Thr-Ser-Asn-Pro-Pro-His-Gly-Leu-Pro-Ser-Thr-Cys.

14. The protein according to claim 5, wherein said protein contains an epitope of HAV coupled to a non HAV particle forming protein to form a complex, such that the complex is capable of forming a particle immunogenic against HAV infection.

15. The protein according to Claim 14 wherein the epitope is derived from amino acids 445-657 or 792-848 of the HAV polypeptide sequence.

16. An oligonucleotide useful for detecting the presence of HAV in a biological sample which comprises a DNA sequence derived from the nucleotide sequence in Figure 1; the oligonucleotide being a fragment according to claim 3.

17. A protein according to any one of Claims 5, 11 and 13-15 for use in a vaccine effective against Hepatitis A virus.

18. An expression system for a deleted HAV virion which comprises a nucleotide sequence encoding the P1 precursor protein as shown in Figure 1, said nucleotide sequence operably linked to suitable control sequences, and transformed into a host cell capable of producing a protease effective in cleaving said P1 precursor to obtain a deleted HAV virion.

19. The expression system of claim 18 wherein said protease is encoded by the 3C HAV region of Figure 1.

20. An expression system according to claim 18 wherein said deleted virion is encoded by a DNA corresponding to nucleotides 734-5767 of the sequence represented in Figure 1.

21. Protein produced by an expression system according to any one of claims 18 to 20.

22. An expression system according to claim 3 wherein said nucleotide sequence encodes VP1, VP2 or VP3.

23. A process for obtaining protein comprising HAV sequences which comprises culturing cells according to claim 4 or claim 10, or cells containing an expression system according to claim 18, and recovering the protein thus obtained.

**Revendications**
**Revendications pour les Etats contractants suivants : CH, DE, FR, GB, LI, NL**

1. Séquence nucléotidique sensiblement identique à celle du génome HAV telle que représentée à la figure 1.

2. Séquence nucléotidique codant pour un polypeptide viral sensiblement identique à celui qui est codé par la séquence génomique HAV représentée à la figure 1.

3. Système d'expression recombinant comprenant une partie codante dérivée de la séquence selon la revendication 1 liée de façon opératoire à une séquence de contrôle compatible avec un hôte désiré; la partie codante étant n'importe quelle partie autre que les fragments sensiblement identiques aux nucléotides 328-3305, 2074-3047, 1812-3305, 1969-3047, 1806-3305, 1808-1834, 2207-2239, 2288-2323, 2324-2413, 2474-2530, 2675-2716 ou 23-3295 de la séquence représentée à la figure 1.

4. Cellules hôtes recombinantes transformées par le système d'expression selon la revendication 3.

5. Protéine produite par les cellules selon la revendication 4.

6. Système selon la revendication 3 qui comprend en outre, contiguë à ladite partie codante, et dans un cadre de lecture avec elle, une séquence nucléotidique fusionnée codant pour une protéine non codée par HAV ou une de ses parties.

7. Système d'expression recombinant comprenant une partie codante dérivée de la séquence selon la revendication 1 liée de façon opératoire à une séquence de contrôle compatible avec un hôte désiré qui inclut en outre, en amont de ladite partie codante, et séparée par un codon d'arrêt, une séquence nucléotidique fusionnée codant pour une protéine non codée par HAV ou une de ses parties.

8. Système selon la revendication 6 ou 7 dans lequel la séquence d'ADN fusionnée code pour la superoxyde dismutase humaine ou une de ses parties.

**9.** Système selon les revendications 6 ou 7 dans lequel la séquence d'ADN fusionnée code pour une protéine non formatrice de particules HAV.

**10.** Cellules hôtes recombinantes transformées par le système selon la revendication 6 ou 7.

**11.** Protéine produite par les cellules selon la revendication 10.

**12.** Système selon la revendication 3 dans lequel la partie codante est la séquence nucléotidique selon la revendication 1 située entre les positions nucléotidiques choisies dans le groupe constitué par 2072-3201; 1182-3201; et 734-3201.

**13.** Peptide antigénique comprenant une séquence dérivée de la séquence selon la revendication 1 liée de façon covalente par le groupement C-terminal à une cystéine, choisie dans le groupe:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; et

Ser-Ser-Thr-Ser-Asn-Pro-Pro-His-Gly-Leu-Pro-Ser-Thr-Cys.

**14.** Protéine selon la revendication 5, dans laquelle ladite protéine contient un épitope de HAV couplé à une protéine non formatrice de particules HAV pour former un complexe, de manière que le complexe soit capable de former une particule immunogène contre l'infection par HAV.

**15.** Protéine selon la revendication 14 dans laquelle l'épitope est dérivé des acides aminés 445-657 ou 792-848 de la séquence polypeptidique de HAV.

**16.** Oligonucléotide utile pour détecter la présence de HAV dans un échantillon biologique qui comprend une séquence d'ADN dérivée de la séquence nucléotidique à la figure 1; l'oligonucléotide étant un fragment selon la revendication 3.

**17.** Protéine selon l'une quelconque des revendications 5, 11 et 13-15 destinée à l'utilisation dans un vaccin efficace contre le virus de l'hépatite A.

**18.** Système d'expression pour un virion de HAV délété qui comprend une séquence nucléotidique codant pour la protéine précurseur P1 telle que représentée à la figure 1, ladite séquence nucléotidique étant liée de façon opératoire à des séquences de contrôle appropriées, et transformée dans une cellule hôte capable de produire une protéase efficace pour cliver ledit précurseur P1 afin d'obtenir un virion de HAV délété.

**19.** Système d'expression selon la revendication 18 dans lequel ladite protéase est codée par la région 3C de HAV à la figure 1.

**20.** Système d'expression selon la revendication 18 dans lequel ledit virion délété est codé par un ADN correspondant aux nucléotides 734-5767 de la séquence représentée à la figure 1.

**21.** Protéine produite par un système d'expression selon l'une quelconque des revendications 18 à 20.

**22.** Système d'expression selon la revendication 3 dans lequel ladite séquence nucléotidique code pour la

VP2.

**23.** Procédé d'obtention d'une protéine comprenant des séquences de HAV dans lequel on cultive des cellules selon la revendication 4 ou la revendication 10, ou des cellules contenant un système d'expression selon la revendication 18, et on recueille la protéine ainsi obtenue.

**Revendications pour les Etats contractants suivants : AT, BE, LU, SE**

**1.** Séquence nucléotidique sensiblement identique à celle du génome HAV telle que représentée à la figure 1.

**2.** Séquence nucléotidique codant pour un polypeptide viral sensiblement identique à celui qui est codé par la séquence génomique HAV représentée à la figure 1.

**3.** Système d'expression recombinant comprenant une partie codante dérivée de la séquence selon la revendication 1 liée de façon opératoire à une séquence de contrôle compatible avec un hôte désiré; la partie codante étant n'importe quelle partie autre que les fragments sensiblement identiques aux nucléotides 23-3295 de la séquence représentée à la figure 1.

**4.** Cellules hôtes recombinantes transformées par le système d'expression selon la revendication 3.

**5.** Protéine produite par les cellules selon la revendication 4.

**6.** Système selon la revendication 3 qui comprend en outre, contiguë à ladite partie codante, et dans un cadre de lecture avec elle, une séquence nucléotidique fusionnée codant pour une protéine non codée par HAV ou une de ses parties.

**7.** Système selon la revendication 3 qui comprend en outre, en amont de ladite partie codante, et séparée par un codon d'arrêt, une séquence nucléotidique fusionnée codant pour une protéine non codée par HAV ou une de ses parties.

**8.** Système de la revendication 6 ou 7 dans lequel la séquence d'ADN fusionnée code pour la superoxyde dismutase humaine ou une de ses parties.

**9.** Système selon les revendications 6 ou 7 dans lequel la séquence d'ADN fusionnée code pour une protéine non formatrice de particules HAV.

**10.** Cellules hôtes recombinantes transformées par le système selon les revendications 6 ou 7.

**11.** Protéine produite par les cellules selon la revendication 10.

**12.** Système selon la revendication 3 dans lequel la partie codante est la séquence nucléotidique selon la revendication 1 située entre les positions nucléotidiques choisies dans le groupe constitué par 2072-3201; 1182-3201; et 734-3201.

**13.** Peptide antigénique comprenant une séquence, dérivée de la séquence selon la revendication 1, liée de façon covalente par le groupement C-terminal à une cystéine, choisie dans le groupe:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; et

Ser-Ser-Thr-Ser-Asn-Pro-Pro-His-Gly-Leu-Pro-Ser-Thr-Cys.

**14.** Protéine selon la revendication 5, dans laquelle ladite protéine contient un épitope de HAV couplé à une protéine non formatrice de particules HAV pour former un complexe, de manière que le complexe soit capable de former une particule immunogène contre l'infection par HAV.

**15.** Protéine selon la revendication 14 dans laquelle l'épitope est dérivé des acides aminés 445-657 ou 792-848 de la séquence polypeptidique de HAV.

**16.** Oligonucléotide utile pour détecter la présence de HAV dans un échantillon biologique qui comprend une séquence d'ADN dérivée de la séquence nucléotidique à la figure I; l'oligonucléotide étant autre que le fragment sensiblement identique aux nucléotides 23-3295 de la séquence représentée à la figure 1.

**17.** Protéine selon l'une quelconque des revendications 5, 11 et 13-15 destinée à l'utilisation dans un vaccin efficace contre le virus de l'hépatite A.

**18.** Système d'expression d'un virion de HAV délété qui comprend une séquence nucléotidique codant pour la protéine précurseur P1 telle que représentée à la figure 1, ladite séquence nucléotidique étant liée de façon opératoire à des séquences de contrôle appropriées, et transformée dans une cellule hôte capable de produire une protéase efficace pour cliver ledit précurseur P1 afin d'obtenir un virion de HAV délété.

**19.** Système d'expression de la revendication 18 dans lequel ladite protéase est codée par la région 3C de HAV à la figure 1.

**20.** Système d'expression selon la revendication 18 dans lequel ledit virion délété est codé par un ADN correspondant aux nucléotides 734-5767 de la séquence représentée à la figure 1.

**21.** Protéine produite par un système d'expression selon l'une quelconque des revendications 18 à 20.

**22.** Système d'expression qui comprend une séquence nucléotidique codant pour une protéine choisie dans le groupe constitué par VP1, VP2 et VP3 liée de façon opératoire à des séquences de contrôle appropriées.

**23.** Procédé d'obtention d'une protéine comprenant des séquences de HAV dans lequel on cultive des cellules selon la revendication 4 ou la revendication 10, ou des cellules contenant un système d'expression selon la revendication 18, et on recueille la protéine ainsi obtenue.

**Revendications pour l'Etat contractant suivant : IT**

**1.** Séquence nucléotidique sensiblement identique à celle du génome HAV telle que représentée à la figure 1.

**2.** Séquence nucléotidique codant pour un polypeptide viral sensiblement identique à celui codé par la

séquence génomique HAV représentée à la figure 1.

**3.** Système d'expression recombinant comprenant une partie codante dérivée de la séquence selon la revendication 1 liée de façon opératoire à une séquence de contrôle compatible avec un hôte désiré; la partie codante étant n'importe quelle partie autre que les fragments sensiblement identiques aux nucléotides 328-3305, 2074-3047, 1812-3305, 1969-3047, 1806-3305, 1808-1834, 2207-2239, 2288-2323, 2324-2413, 2474-2530 ou 2675-2716 de la séquence représentée à la figure 1.

**4.** Cellules hôtes recombinantes transformées par le système d'expression selon la revendication 3.

**5.** Protéine produite par les cellules selon la revendication 4.

**6.** Système selon la revendication 3 qui comprend en outre, contiguë à ladite partie codante, et dans un cadre de lecture avec elle, une séquence nucléotidique fusionnée codant pour une protéine non codée par HAV ou une de ses parties.

**7.** Système d'expression recombinant comprenant une partie codante dérivée de la séquence selon la revendication 1 liée de façon opératoire à une séquence de contrôle compatible avec un hôte désiré qui inclut en outre, en amont de ladite partie codante, et séparée par un codon d'arrêt, une séquence nucléotidique fusionnée codant pour une protéine non codée par HAV ou une de ses parties.

**8.** Système selon la revendication 6 ou 7 dans lequel la séquence d'ADN fusionnée code pour la superoxyde dismutase humaine ou une de ses parties.

**9.** Système selon les revendications 6 ou 7 dans lequel la séquence d'ADN fusionnée code pour une protéine non formatrice de particules HAV.

**10.** Cellules hôtes recombinantes transformées par le système selon la revendication 6 ou 7.

**11.** Protéine produite par les cellules selon la revendication 10.

**12.** Système selon la revendication 3 dans lequel la partie codante est la séquence nucléotidique selon la revendication 1 située entre les positions nucléotidiques choisies dans le groupe constitué par 2072-3201; 1182-3201; et 734-3201.

**13.** Peptide antigénique comprenant une séquence, dérivée de la séquence selon la revendication 1, liée de façon covalente par le groupement C-terminal à une cystéine, choisie dans le groupe:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; et

Ser-Ser-Thr-Ser-Asn-Pro-Pro-His-Gly-Leu-Pro-Ser-Thr-Cys.

**14.** Protéine selon la revendication 5, dans laquelle ladite protéine contient un épitope de HAV couplé à une protéine non formatrice de particules HAV pour former un complexe, de manière que le complexe soit capable de former une particule immunogène contre l'infection par HAV.

**15.** Protéine selon la revendication 14 dans laquelle l'épitope est dérivé des acides aminés 445-657 ou

792-848 de la séquence polypeptidique de HAV.

16. Oligonucléotide utile pour détecter la présence de HAV dans un échantillon biologique qui comprend une séquence d'ADN dérivée de la séquence nucléotidique à la figure 1; l'oligonucléotide étant un fragment selon la revendication 3.

17. Protéine selon l'une quelconque des revendications 5, 11 et 13-15 destinée à l'utilisation dans un vaccin efficace contre le virus de l'hépatite A.

18. Système d'expression pour un virion de HAV délété qui comprend une séquence nucléotidique codant pour la protéine précurseur P1 telle que représentée à la figure 1, ladite séquence nucléotidique étant liée de façon opératoire à des séquences de contrôle appropriées, et transformée dans une cellule hôte capable de produire une protéase efficace pour cliver ledit précurseur P1 afin d'obtenir un virion de HAV délété.

19. Système d'expression selon la revendication 18 dans lequel ladite protéase est codée par la région 3C de HAV à la figure 1.

20. Système d'expression selon la revendication 18 dans lequel ledit virion délété est codé par un ADN correspondant aux nucléotides 734-5767 de la séquence représentée à la figure 1.

21. Protéine produite par un système d'expression selon l'une quelconque dese revendications 18 à 20.

22. Système d'expression selon la revendication 3 dans lequel ladite séquence nucléotidique code pour la VP1, la VP2 ou la VP3.

23. Procédé d'obtention d'une protéine comprenant des séquences de HAV dans lequel on cultive des cellules selon la revendication 4 ou la revendication 10, ou des cellules contenant un système d'expression selon la revendication 18, et on recueille la protéine ainsi obtenue.

**Patentansprüche**
**Patentansprüche für folgende Vertragsstaaten : CH, DE, FR, GB, LI, NL**

1. Nukleotidsequenz, im wesentlichen identisch mit der des HAV-Genoms, wie in Figur 1 dargestellt.

2. Nukleotidsequenz, codierend für ein virales Polypeptid, das im wesentlichen mit dem identisch ist, das von der HAV-Genomsequenz, wie in Figur 1 gezeigt, codiert wird.

3. Rekombinantes Expressionssystem, umfassend einen codierenden Teil, abgeleitet von der Sequenz nach Anspruch 1, in ausführbarer Weise geknüpft an eine mit dem gewünschten Wirt kompatible Kontrollsequenz, wobei der codierende Teil jeder beliebige Teil außer Fragmente, die im wesentlichen identisch mit den Nukleotiden 328-3305, 2074-3047, 1812-3305, 1969-3047, 1806-3305, 1808-1834, 2207-2239, 2288-2323, 2324-2413, 2474-2530, 2675-2716 oder 23-3295 der Sequenz nach Figur 1 sind, ist.

4. Rekombinante Wirtszellen, transformiert mit dem Expressionssystem nach Anspruch 3.

5. Protein, gebildet von den Zellen nach Anspruch 4.

6. System nach Anspruch 3, dadurch **gekennzeichnet,** daß es weiterhin, angrenzend an den codierenden Teil und im Leseraster damit eine fusionierte Nukleotidsequenz, die für ein nicht-HAV-codiertes Protein oder einen Teil davon codiert, enthält.

7. Rekombinantes Expressionssystem, umfassend einen codierenden Teil, abgeleitet von der Sequenz nach Anspruch 1, in ausführbarer Weise gebunden an eine mit dem gewünschten Wirt verträgliche Kontrollsequenz, enthaltend weiterhin stromaufwärts des codierenden Teils und getrennt durch ein Stopcodon, eine fusionierte Nukleotidsequenz, die für ein nicht-HAV-codiertes Protein oder einen Teil davon codiert.

8. System nach Anspruch 6 oder 7, dadurch **gekennzeichnet,** daß die fusionierte DNA-Sequenz für menschliche Superoxid-Dismutase oder einen Teil davon codiert.

9. System nach den Ansprüchen 6 oder 7, dadurch **gekennzeichnet,** daß die fusionierte DNA-Sequenz für ein nicht-HAV-Teilchen-bildendes Protein codiert.

10. Rekombinante Wirtszellen, transformiert mit dem System nach Anspruch 6 oder 7.

11. Protein, gebildet von den Zellen nach Anspruch 10.

12. System nach Anspruch 3, dadurch **gekennzeichnet,** daß der codierende Teil die Nukleotidsequenz nach Anspruch 1 zwischen den Nukleotidpositionen, ausgewählt aus der Gruppe bestehend aus 2072-3201, 1182-3201 und 734-3201, ist.

13. Antigenes Peptid, umfassend eine Sequenz, abgeleitet von der Sequenz nach Anspruch 1, in covalenter Bindung an den C-Terminus an Cystein, ausgewählt aus der Gruppe:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; und

Ser-Ser-Thr-Ser-Asn-Pro-Pro-His-Gly-Leu-Pro-Ser-Thr-Cys.

14. Protein nach Anspruch 5, dadurch **gekennzeichnet,** daß das Protein ein Epitop von HAV in Kopplung an ein nicht-HAV-Teilchen-bildendes Protein, unter Bildung eines Komplexes enthält, so daß der Komplex in der Lage ist, ein Teilchen zu bilden, das gegen eine HAV-Infektion immunogen ist.

15. Protein nach Anspruch 14, dadurch **gekennzeichnet,** daµ das Epitop von den Aminosäuren 445-657 oder 792-848 der HAV-Polypeptidsequenz abgeleitet ist.

16. Oligonukleotid, geeignet zur Detektion auf die Anwesenheit von HAV in einer biologischen Probe, umfassend eine DNA-Sequenz, abgeleitet von der Nukleotidsequenz nach Figur 1, wobei das Oligonukleotid ein Fragment nach Anspruch 3 ist.

17. Protein nach einem der Ansprüche 5, 11 und 13 bis 15 zur Verwendung in einem Impfstoff, der gegen das Hepatitis-A-Virus wirksam ist.

18. Expressionssystem für ein deletiertes HAV-Virion, umfassend eine Nukleotidsequenz, codierend für das P1-Precursor-Protein, wie in Figur 1 gezeigt, wobei die Nukleotidsequenz in ausführbarer Weise an geeignete Kontrollsequenzen gebunden ist und in eine Wirtszelle transformiert ist, die in der Lage ist, eine Protease zu erzeugen, die wirksam den P1-Precursor unter Erhalt eines deletierten HAV-Virions spaltet.

19. Expressionssystem nach Anspruch 18, dadurch **gekennzeichnet,** daß die Protease von der 3C HAV-Region der Figur 1 codiert ist.

20. Expressionssystem nach Anspruch 18, dadurch **gekennzeichnet,** daß das deletierte Virion von einer DNA, entsprechend den Nukleotiden 734-5767 der Sequenz nach Figur 1 codiert ist.

**21.** Protein, gebildet von einem Expressionssystem nach einem der Ansprüche 18 bis 20.

**22.** Expressionssystem nach Anspruch 3, dadurch **gekennzeichnet,** daß die Nukleotidsequenz für VP2 codiert.

**23.** Verfahren zur Herstellung eines Proteins, umfassend HAV-Sequenzen, dadurch **gekennzeichnet,** daß man Zellen nach Anspruch 4 oder Anspruch 10 oder Zellen, die ein Expressionssystem nach Anspruch 18 enthalten, kultiviert und das so erhaltene Protein gewinnt.

**Patentansprüche für folgende Vertragsstaaten : AT, BE, LU, SE**

**1.** Nukleotidsequenz, im wesentlichen identisch mit der des HAV-Genoms, wie in Figur 1 dargestellt.

**2.** Nukleotidsequenz, codierend für ein virales Polypeptid, das im wesentlichen mit dem identisch ist, das von der HAV-Genomsequenz, wie in Figur 1 gezeigt, codiert wird.

**3.** Rekombinantes Expressionssystem, umfassend einen codierenden Teil, abgeleitet von der Sequenz nach Anspruch 1, in ausführbarer Weise geknüpft an eine mit dem gewünschten Wirt kompatible Kontrollsequenz, wobei der codierende Teil ein anderer als die Fragmente, die im wesentlichen den Nukleotiden 23-3295 der Sequenz nach Figur 1 identisch sind, ist.

**4.** Rekombinante Wirtszellen, transformiert mit dem Expressionssystem nach Anspruch 3.

**5.** Protein, gebildet von den Zellen nach Anspruch 4.

**6.** System nach Anspruch 3, dadurch **gekennzeichnet,** daß es weiterhin, angrenzend an den codierenden Teil und im Leseraster damit eine fusionierte Nukleotidsequenz, die für ein nicht-HAV-codiertes Protein oder einen Teil davon codiert, enthält.

**7.** System nach Anspruch 3, dadurch **gekennzeichnet,** daß es weiterhin stromaufwärts des codierenden Teils und abgetrennt durch ein Stopcodon eine fusionierte Nukleotidsequenz, die für ein nicht-HAV-codiertes Protein oder einen Teil davon codiert, umfaßt.

**8.** System nach Anspruch 6 oder 7, dadurch **gekennzeichnet,** daß die fusionierte DNA-Sequenz für Human-Superoxid-Dismutase oder einen Teil davon codiert.

**9.** System nach Anspruch 6 oder 7, dadurch **gekennzeichnet,** daß die fusionierte DNA-Sequenz für ein nicht-HAV-Teilchen-bildendes Protein codiert.

**10.** Rekombinante Wirtszellen, transformiert mit dem System nach Anspruch 6 oder 7.

**11.** Protein, gebildet von den Zellen nach Anspruch 10.

**12.** System nach Anspruch 3, dadurch **gekennzeichnet,** daß der codierende Teil die Nukleotidsequenz nach Anspruch 1 zwischen die Nukleotidpositionen, ausgewählt aus der Gruppe bestehend aus 2072-3201, 1182-3201 und 734-3201, ist.

**13.** Antigenes Peptid, umfassend eine Sequenz, abgeleitet von der Sequenz nach Anspruch 1 in covalenter Bindung an den C-Terminus an Cystein, ausgewählt aus der Gruppe

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; und

Ser-Ser-Thr-Ser-Asn-Pro-Pro-His-Gly-Leu-Pro-Ser-Thr-Cys.

14. Protein nach Anspruch 5, dadurch **gekennzeichnet,** daß das Protein ein Epitop von HAV in Kopplung an ein nicht-HAV-Teilchen-bildendes Protein, unter Bildung eines Komplexes enthält, so daß der Komplex in der Lage ist, ein Teilchen zu bilden, das gegen eine HAV-Infektion immunogen ist.

15. Protein nach Anspruch 14, dadurch **gekennzeichnet,** daß das Epitop von den Aminosäuren 445-657 oder 792-848 der HAV-Polypeptidsequenz abgeleitet ist.

16. Oligonukleotid, geeignet zur Detektion auf die Anwesenheit von HAV in einer biologischen Probe, umfassend eine DNA-Sequenz, abgeleitet von der Nukleotidsequenz in Figur 1, wobei das Oligonukleotid ein anderes ist, als das Fragment das im wesentichen mit den Nukleotiden 23-3295 der Sequenz nach Figur 1 identisch ist.

17. Protein nach einem der Ansprüche 5, 11 und 13 bis 15 zur Verwendung in einem Impfstoff, der wirksam gegen das Hepatitis-A-Virus ist.

18. Expressionssystem für ein deletiertes HAV-Virion, umfassend eine Nukleotidsequenz die für das P1-Precursor-Protein, wie in Figur 1 gezeigt, codiert, wobei die Nukleotidsequenz in ausführbarer Weise an geeignete Kontrollsequenzen gebunden ist und in eine Wirtszelle transformiert ist, die in der Lage ist, eine Protease die den P1-Precursor unter Erhalt eines deletierten HAV-Virions effektiv zu spalten, zu bilden.

19. Expressionssystem nach Anspruch 18, dadurch **gekennzeichnet,** daß die Protease von der 3C HAV-Region nach Figur 1 codiert ist.

20. Expressionssystem nach Anspruch 18, dadurch **gekennzeichnet,** daß das deletierte Virion von einer DNA, entsprechend den Nukleotiden 734-5767 der Sequenz nach Figur 1 codiert ist.

21. Protein, gebildet von einem Expressionssystem nach einem der Ansprüche 18 bis 20.

22. Expressionssystem, umfassend eine Nukleotidsequenz, codierend für ein Protein, ausgewählt aus der Gruppe, bestehend aus VP1, VP2 und VP3 in ausführbarer Weise gebunden an geeignete Kontrollsequenzen.

23. Verfahren zur Herstellung eines Proteins, umfassend HAV-Sequenzen, dadurch **gekennzeichnet,** daß man Zellen nach Anspruch 4 oder 10 oder Zellen, die ein Expressionssystem nach Anspruch 18 enthalten, kultiviert und das so erhaltene Protein gewinnt.

**Patentansprüche für folgenden Vertragsstaat : IT**

1. Nukleotidsequenz, im wesentlichen identisch mit der des HAV-Genoms, wie in Figur 1 dargestellt.

2. Nukleotidsequenz, codierend für ein virales Polypeptid, das im wesentlichen mit dem identisch ist, das von der HAV-Genomsequenz, wie in Figur 1 gezeigt, codiert wird.

3. Rekombinantes Expressionssystem, umfassend einen codierenden Teil, abgeleitet von der Sequenz nach Anspruch 1, in ausführbarer Weise geknüpft an eine mit dem gewünschten Wirt kompatible

Kontrollsequenz, wobei der codierende Teil jeder beliebige Teil außer Fragmente, die im wesentlichen identisch mit den Nukleotiden 328-3305, 2074-3047, 1812-3305, 1969-3047, 1806-3305, 1808-1834, 2207-2239, 2288-2323, 2324-2413, 2474-2530 oder 2675-2716 der Sequenz nach Figur 1 sind, ist.

**4.** Rekombinante Wirtszellen, transformiert mit dem Expressionssystem nach Anspruch 3.

**5.** Protein, gebildet von den Zellen nach Anspruch 4.

**6.** System nach Anspruch 3, dadurch **gekennzeichnet,** daß es weiterhin, angrenzend an den codierenden Teil und im Leseraster damit, eine fusionierte Nukleotidsequenz, die für ein nicht-HAV-codiertes Protein oder einen Teil davon codiert, enthält.

**7.** Rekombinantes Expressionssystem, umfassend einen codierenden Teil, abgeleitet von der Sequenz nach Anspruch 1, in ausführbarer Weise gebunden an eine mit dem gewünschten Wirt verträgliche Kontrollsequenz, enthaltend weiterhin stromaufwärts des codierenden Teils und getrennt durch ein Stopcodon, eine fusionierte Nukleotidsequenz, die für nicht-HAV-codiertes Protein oder einen Teil davon codiert.

**8.** System nach Anspruch 6 oder 7, dadurch **gekennzeichnet,** daß die fusionierte DNA-Sequenz für menschliche Superoxid-Dismutase oder einen Teil davon codiert.

**9.** System nach den Ansprüchen 6 oder 7, dadurch **gekennzeichnet,** daß die fusionierte DNA-Sequenz für ein nicht-HAV-Teilchen-bildendes Protein codiert.

**10.** Rekombinante Wirtszellen, transformiert mit dem System nach Anspruch 6 oder 7.

**11.** Protein, gebildet von den Zellen nach Anspruch 10.

**12.** System nach Anspruch 3, dadurch **gekennzeichnet,** daß der codierende Teile die Nukleotidsequenz nach Anspruch 1 zwischen den Nukleotidpositionen, ausgewählt aus der Gruppe bestehend aus 2072-3201, 1182-3201 und 734-3201, ist.

**13.** Antigenes Peptid, umfassend eine Sequenz, abgeleitet von der Sequenz nach Anspruch 1, in covalenter Bindung an den C-Terminus an Cystein, ausgewählt aus der Gruppe:

Pro-Glu-Leu-Lys-Pro-Gly-Glu-Ser-Arg-His-Thr-Ser-Asp-His-Met-Ser-Cys;

Thr-Phe-Asn-Ser-Asn-Asn-Lys-Glu-Tyr-Thr-Phe-Pro-Cys; und

Ser-Ser-Thr-Ser-Asn-Pro-Pro-His-Gly-Leu-Pro-Ser-Thr-Cys.

**14.** Protein nach Anspruch 5, dadurch **gekennzeichnet,** daß das Protein ein Epitop von HAV in Kopplung an ein nicht-HAV-Teilchen-bildendes Protein, unter Bildung eines Komplexes enthält, so daß der Komplex in der Lage ist, ein Teilchen zu bilden, das gegen eine HAV-Infektion immunogen ist.

**15.** Protein nach Anspruch 14, dadurch **gekennzeichnet,** daß das Epitop von den Aminosäuren 445-657 oder 792-848 der HAV-Polypeptidsequenz abgeleitet ist.

**16.** Oligonukleotid, geeignet zur Detektion auf die Anwesenheit von HAV in einer biologischen Probe, umfassend eine DNA-Sequenz, abgeleitet von der Nukleotidsequenz nach Figur 1, wobei das Oligonukleotid ein Fragment nach Anspruch 3 ist.

**17.** Protein nach einem der Ansprüche 5, 11 und 13 bis 15 zur Verwendung in einem Impfstoff, der gegen das Hepatitis-A-Virus wirksam ist.

**18.** Expressionssystem für ein deletiertes HAV-Virion, umfassend eine Nukleotidsequenz, codierend für das P1-Precursor-Protein, wie in Figur 1 gezeigt, wobei die Nukleotidsequenz in ausführbarer Weise an geeignete Kontrollsequenzen gebunden ist und in eine Wirtszelle transformiert ist, die in der Lage ist, eine Protease zu erzeugen, die wirksam den P1-Precursor unter Erhalt eines deletierten HAV-Virions spaltet.

**19.** Expressionssystem nach Anspruch 18, dadurch **gekennzeichnet,** daß die Protease von der 3C HAV-Region der Figur 1 codiert ist.

**20.** Expressionssystem nach Anspruch 18, dadurch **gekennzeichnet,** daß das deletierte Virion von einer DNA, entsprechend den Nukleotiden 734-5767 der Sequenz nach Figur 1 codiert ist.

**21.** Protein, gebildet von einem Expressionssystem nach einem der Ansprüche 18 bis 20.

**22.** Expressionssystem nach Anspruch 3, dadurch **gekennzeichnet,** daß die Nukleotidsequenz für VP1, VP2 oder VP3 codiert.

**23.** Verfahren zur Herstellung eines Proteins, umfassend HAV-Sequenzen, dadurch **gekennzeichnet,** daß man Zellen nach Anspruch 4 oder Anspruch 10 oder Zellen, die ein Expressionssystem nach Anspruch 18 enthalten, kultiviert und das so erhaltene Protein gewinnt.

```
TTCAAGAGGGGTCTCCGGAGGTTTCCGGAGGCCCTCTTGGAAGTCCATGGTGAGGGGACTTGATACCTCACCGCCGGTTTGGCTAGGGTATAGGGTAAATTTCCCTTTCCCTGTCCCTCCCTTATTTCCCTTTGTTTTGGTTGTAAAT (147)
ATTAATTCCTGCAGCTTCAGCGTTCTTTAATCTGTTTCTCTATAAGAACACTCAATTTTTCACGCTTTCTCTCTTCTTTCTTCCAGGGCTCTCCCCTTGCCCTAGGCTCTGCCCGTTGCGCCCGCCGGGCTCAACTCCATGATTAGCA (294)
TGGAGCTTGTAGCAGTCTAAATTGCGGACGCAGATGTTTGGGACGTCACCTTGCAGTGTTAACTTGCCTCTCATGAACCTCTTTCATCTTCCACAAGGGGTAGCCTACGGGTGAAACCTCTTAGGCTAATACTTCTATGAAGAGATGC (441)
TTTGGATAGGCTAACACGCGCGGATATTGGTGAGTTGTTAAGACAAAAACCATTCAACGCCCGAGGACTGGCTCTCATCCAGTGGATGGATTGAGTGGATTGATTGTCAGGGCTGTCTCTAGGTTTAATCTCAGACCTCTCTGTGCT (588)
TAGGGCAAACACCATTTGGCCTTAAATGGGATCCTGTGAGACGGGGTCCCTCCATTCACAGCTGGACTGTTCTTTGCGGGCCTTATGTGGTGTTTGCCTCTGAGGTACTCAGGGGCATTTAGGTTTTTCCTCATTCTTAAACAATA (733)

P1,1A
1                                     10                                      20                                      30
Met Asn Met Ser Lys Gln Gly Ile Phe Gln Thr Val Gly Ser Gly Leu Asp His Ile Leu Ser Leu Ala Asp Ile Glu Glu Glu Gln Met Ile Gln Ser Val Asp Arg Thr
ATG AAT ATG TCC AAA CAA GGA ATT TTC CAG ACT GTT GGG AGT GGC CTT GAC CAC ATC CTG TCT TTG GCA GAT ATT GAG GAA GAG CAA ATG ATT CAG TCC GTT GAT AGG ACT (844)

        40                                      50                                      60                                      70
Ala Val Thr Gly Ala Ser Tyr Phe Thr Ser Val Asp Gln Ser Ser Val His Thr Ala Glu Val Gly Ser His Gln Ile Glu Pro Leu Lys Thr Ser Val Asp Lys Pro Gly
GCA GTG ACT GGA GCT TCT TAC TTC ACT TCT GTG GAC CAA TCT TCA GTT CAT ACT GCT GAG GTT GGC TCA CAT CAA ATT GAA CCT TTG AAA ACC TCT GTT GAT AAA CCT GGT (955)

                    80                                      90                                      100                                     110
Ser Lys Lys Thr Gln Gly Glu Lys Phe Phe Leu Ile His Ser Ala Asp Trp Leu Thr Thr His Ala Leu Phe His Glu Val Ala Lys Leu Asp Val Val Lys Leu Leu Tyr
TCT AAG AAA ACT CAG GGG GAA AAG TTT TTC CTG ATT CAT TCT GCT GAT TGG CTC ACT ACA CAT GCT CTC TTT CAT GAA GTT GCA AAA TTG GAT GTG GTG AAA CTA CTG TAT (1066)

                                120                                     130                                     140
Asn Glu Gln Phe Ala Val Gln Gly Leu Leu Arg Tyr His Thr Tyr Ala Arg Phe Gly Ile Glu Ile Gln Val Gln Ile Asn Pro Thr Pro Phe Gln Gln Gly Gly Leu Ile
AAT GAG CAG TTT GCC GTC CAA GGT TTG TTG AGA TAC CAT ACA TAT GCA AGA TTT GGC ATT GAG ATT CAA GTT CAG ATA AAT CCC ACA CCC TTT CAG CAA GGA GGA CTA ATT (1177)

    150                                     160                                     170                                     180
Cys Ala Met Val Pro Gly Asp Gln Ser Tyr Gly Ser Ile Ala Ser Leu Thr Val Tyr Pro His Gly Leu Leu Asn Cys Asn Ile Asn Asn Val Val Arg Ile Lys Val Pro
TGT GCC ATG GTT CCT GGT GAC CAA AGT TAT GGT TCA ATA GCA TCC TTG ACT GTT TAT CCT CAT GGT CTG TTA AAT TGC AAT ATC AAC AAT GTA GTT AGA ATA AAG GTT CCA (1288)

                190                                     200                                     210                                     220
Phe Ile Tyr Thr Arg Gly Ala Tyr His Phe Lys Asp Pro Gln Tyr Pro Val Trp Glu Leu Thr Ile Arg Val Trp Ser Glu Leu Asn Ile Gly Thr Gly Thr Ser Ala Tyr
TTT ATT TAT ACT AGA GGT GCT TAT CAT TTT AAA GAT CCA CAG TAC CCA GTT TGG GAA TTG ACA ATC AGA GTT TGG TCA GAG TTG AAT ATT GGA ACA GGA ACT TCA GCT TAC (1399)

                            230                                     240                     1B                  250
Thr Ser Leu Asn Val Leu Ala Arg Phe Thr Asp Leu Glu Leu His Gly Leu Thr Pro Leu Ser Thr Gln Met Met Arg Asn Glu Phe Arg Val Ser Thr Thr Glu Asn Val
ACT TCA CTC AAT GTT TTA GCT AGG TTT ACA GAT TTG GAG TTG CAT GGA TTA ACT CCT CTT TCT ACA CAG ATG ATG AGA AAT GAA TTT AGG GTC AGT ACT ACT GAA AAT GTT (1510)

260                                     270                                     280                                     290
Val Asn Leu Ser Asn Tyr Glu Asp Ala Arg Ala Lys Met Ser Phe Ala Leu Asp Gln Glu Asp Trp Lys Ser Asp Pro Ser Gln Gly Gly Gly Ile Lys Ile Thr His Phe
GTA AAT TTG TCA AAT TAT GAA GAT GCA AGG GCA AAA ATG TCT TTT GCT TTG GAT CAG GAA GAT TGG AAG TCT GAT CCT TCC CAA GGT GGT GGA ATT AAA ATT ACT CAT TTT (1621)

            300                                     310                                     320                                     330
Thr Thr Trp Thr Ser Ile Pro Thr Leu Ala Ala Gln Phe Pro Phe Asn Ala Ser Asp Ser Val Gly Gln Gln Ile Lys Val Ile Pro Val Asp Pro Tyr Phe Phe Gln Met
ACT ACC TGG ACA TCC ATT CCA ACC TTA GCT GCT CAG TTT CCA TTT AAT GCT TCA GAT TCA GTT GGA CAA CAA ATT AAA GTT ATT CCA GTG GAC CCA TAC TTT TTC CAA ATG (1732)

                        340                                     350                                     360                                     370
Thr Asn Thr Asn Pro Asp Gln Lys Cys Ile Thr Ala Leu Ala Ser Ile Cys Gln Met Phe Cys Phe Trp Arg Gly Asp Leu Val Phe Asp Phe Gln Val Phe Pro Thr Lys
ACA AAC ACT AAT CCT GAT CAA AAA TGT ATA ACT GCC TTG GCC TCT ATT TGT CAG ATG TTC TGC TTT TGG AGG GGA GAT CTT GTT TTT GAT TTT CAG GTT TTT CCA ACC AAA (1843)

                                    380                                     390                                     400
Tyr His Ser Gly Arg Leu Leu Phe Cys Phe Val Pro Gly Asn Glu Leu Ile Asp Val Thr Gly Ile Thr Leu Lys Gln Ala Thr Thr Ala Pro Cys Ala Val Met Asp Ile
TAT CAT TCA GGT AGA CTG TTG TTT TGT TTT GTT CCT GGG AAT GAG TTA ATA GAT GTT ACT GGA ATT ACA TTA AAA CAG GCA ACT ACT GCT CCT TGT GCA GTG ATG GAC ATT (1954)

        410                                     420                                     430                                     440
Thr Gly Val Gln Ser Thr Leu Arg Phe Arg Val Pro Trp Ile Ser Asp Thr Pro Tyr Arg Val Asn Arg Tyr Thr Lys Ser Ala His Gln Lys Gly Glu Tyr Thr Ala Ile
ACA GGA GTG CAG TCA ACC TTG AGA TTT CGT GTT CCT TGG ATT TCT GAT ACA CCT TAT CGA GTG AAT AGG TAC ACC AAG TCA GCA CAT CAA AAA GGT GAG TAC ACT GCC ATT (2065)

                    450                                     460                                     470                                     480
Gly Lys Leu Ile Val Tyr Cys Tyr Asn Arg Leu Thr Ser Pro Ser Asn Val Ala Ser His Val Arg Val Asn Val Tyr Leu Ser Ala Ile Asn Leu Glu Cys Phe Ala Pro
GGG AAG CTT ATT GTG TAT TGT TAT AAC AGA CTG ACT TCT CCT TCT AAT GTT GCC TCT CAT GTT AGA GTT AAT GTT TAT CTT TCA GCA ATT AAT TTG GAA TGT TTT GCT CCT (2176)

                                490     1C                              500                                     510
Leu Tyr His Ala Met Asp Val Thr Thr Gln Val Gly Asp Asp Ser Gly Gly Phe Ser Thr Thr Val Ser Thr Glu Gln Asn Val Pro Asp Pro Gln Val Gly Ile Thr Thr
CTT TAC CAT GCT ATG GAT GTT ACT ACA CAG GTT GGA GAT GAT TCA GGA GGT TTC TCA ACA ACA GTT TCT ACA GAG CAG AAT GTT CCT GAT CCC CAA GTT GGG ATA ACA ACC (2287)

    520                                     530                                     540                                     550
Met Arg Asp Leu Lys Gly Lys Ala Asn Arg Gly Lys Met Asp Val Ser Gly Val Gln Ala Pro Arg Gly Ser Tyr Gln Gln Gln Leu Asn Asp Pro Val Leu Ala Lys Lys
ATG AGG GAT TTA AAA GGA AAA GCC AAT AGG GGA AAG ATG GAT GTT TCA GGA GTG CAA GCA CCT CGT GGG AGC TAT CAG CAA CAA TTG AAC GAT CCA GTT TTA GCA AAG AAA (2398)

                560                                     570                                     580                                     590
Val Pro Glu Thr Phe Pro Glu Leu Lys Pro Gly Glu Ser Arg His Thr Ser Asp His Met Ser Ile Tyr Lys Phe Met Gly Arg Ser His Phe Leu Cys Thr Phe Thr Phe
GTA CCT GAG ACA TTT CCT GAA TTG AAG CCT GGA GAG TCC AGA CAT ACA TCA GAT CAC ATG TCT ATT TAT AAA TTC ATG GGA AGG TCT CAT TTT TTG TGC ACT TTT ACT TTC (2509)

                            600                                     610                                     620
Asn Ser Asn Asn Lys Glu Tyr Thr Phe Pro Ile Thr Leu Ser Ser Thr Ser Asn Pro Pro His Gly Leu Pro Ser Thr Leu Arg Trp Phe Phe Asn Leu Phe Gln Leu Tyr
AAT TCA AAT AAT AAA GAG TAC ACA TTT CCA ATA ACC CTG TCT TCC ACT TCT AAT CCT CCT CAT GGT TTA CCA TCA ACA TTA AGG TGG TTC TTC AAT TTG TTT CAG TTG TAT (2620)

630                                     640                                     650                                     660
Arg Gly Pro Leu Asp Leu Thr Ile Ile Ile Thr Gly Ala Thr Asp Val Asp Gly Met Ala Trp Phe Thr Pro Val Gly Leu Ala Val Asp Pro Trp Val Glu Lys Glu Ser
AGA GGA CCA TTG GAT TTA ACA ATT ATA ATC ACA GGA GCC ACT GAT GTG GAT GGT ATG GCC TGG TTT ACT CCA GTG GGC CTT GCT GTC GAC CCT TGG GTG GAA AAG GAG TCA (2731)

            670                                     680                                     690                                     700
Ala Leu Ser Ile Asp Tyr Lys Thr Ala Leu Gly Ala Val Arg Phe Asn Thr Arg Arg Thr Gly Asn Ile Gln Ile Arg Leu Pro Trp Tyr Ser Tyr Leu Tyr Ala Val Ser
GCT TTG TCT ATT GAT TAT AAA ACT GCC CTT GGA GCT GTT AGA TTT AAT ACA AGA AGA ACA GGA AAC ATT CAA ATT AGA TTG CCG TGG TAT TCT TAT TTG TAT GCC GTG TCT (2842)
```

FIGURE 1a

```
                        710                                     720                                     730                                     740
Gly Ala Leu Asp Gly Leu Gly Asp Lys Thr Asp Ser Thr Phe Gly Leu Phe Leu Phe Glu Ile Ala Asn Tyr Asn His Ser Asp Glu Tyr Leu Ser Phe Ser Cys Tyr Leu
GGA GCA CTC GAT GGC TTC GGG GAT AAG ACA GAT TCT ACA TTT GGA TTC TTT CTA TTC GAG ATT GCA AAT TAC AAT CAT TCT GAT GAA TAT TTG TCC TTC AGT TGT TAT TTG (2953)

                                    750                                     760                                     770
Ser Val Thr Glu Gln Ser Glu Phe Tyr Phe Pro Arg Ala Pro Leu Asn Ser Asn Ala Met Leu Ser Thr Glu Ser Met Met Ser Arg Ile Ala Ala Gly Asp Leu Glu Ser
TCT GTC ACA GAG CAA TCA GAG TTC TAT TTT CCT AGA GCT CCA TTA AAT TCA AAT GCT ATG TTG TCC ACT GAA TCC ATG ATG AGT AGA ATT GCA GCT GGA GAC TTG GAG TCA (3064)

        780                                     790                                     800                                     810
Ser Val Asp Asp Pro Arg Ser Glu Glu Asp Arg Arg Phe Glu Ser His Ile Glu Cys Arg Lys Pro Tyr Lys Glu Leu Arg Leu Glu Val Gly Lys Gln Arg Leu Lys Tyr
TCA GTG GAT GAT CCC AGA TCA GAG GAG GAT AGA AGA TTT GAG AGT CAT ATA GAA TGT AGG AAA CCA TAC AAA GAA TTG AGA CTG GAG GTT GGG AAA CAA AGA CTC AAA TAT (3175)

                                                                                        P2,2A
                    820                                     830                                     840                                     850
Ala Gln Glu Glu Leu Ser Asn Glu Val Leu Pro Pro Pro Arg Lys Met Lys Gly Leu Phe Ser Gln Ala Lys Ile Ser Leu Phe Tyr Thr Glu Glu His Glu Ile Met Lys
GCT CAG GAA GAG TTA TCA AAT GAA GTG CTT CCA CCT CCT AGG AAA ATG AAG GGC TTA TTT TCA CAA GCT AAA ATT TCT CTT TTT TAT ACT GAG GAG CAT GAA ATA ATG AAG (3286)

                                860                                     870                                     880
Phe Ser Trp Arg Gly Val Thr Ala Asp Thr Arg Ala Leu Arg Arg Phe Gly Phe Ser Leu Ala Ala Gly Arg Ser Val Trp Thr Leu Glu Met Asp Ala Gly Val Leu Thr
TTT TCT TGG AGA GGA GTG ACT GCT GAT ACT ACC GCT TTG AGA AGA TTT GGA TTC TCT CTG GCT GCT GGT AGA AGT GTG TGG ACT CTT GAA ATG GAT GCT GGA GTT CTT ACT (3397)

    890                                     900                                     910                                     920
Gly Arg Leu Ile Arg Leu Asn Asp Glu Lys Trp Thr Glu Met Lys Asp Asp Lys Ile Val Ser Leu Ile Glu Lys Phe Thr Ser Asn Lys Tyr Trp Ser Lys Val Asn Phe
GGA AGA TTG ATC AGA TTG AAT GAT GAG AAA TGG ACA GAA ATG AAG GAT GAT AAG ATT GTT TCA TTA ATT GAA AAG TTC ACA AGC AAT AAA TAT TGG TCT AAA GTG AAT TTT (3508)

                930                                     940                                     950                                     960
Pro His Gly Met Leu Asp Leu Glu Glu Ile Ala Ala Asn Ser Lys Asp Phe Pro Asn Met Ser Glu Thr Asp Leu Cys Phe Leu Leu His Trp Leu Asn Pro Lys Lys Ile
CCA CAT GGA ATG TTG GAT CTT GAA GAA ATT GCT GCC AAT TCT AAG GAT TTT CCA AAT ATG TCT GAG ACA GAT TTG TGT TTC CTG TTA CAT TGG CTA AAT CCA AAG AAA ATC (3619)

                                                                        2B
                            970                                     980                                     990
Asn Leu Ala Asp Arg Met Leu Gly Leu Ser Gly Val Gln Glu Ile Lys Glu Gln Gly Val Gly Leu Ile Ala Glu Cys Arg Thr Phe Leu Asp Ser Ile Ala Gly Thr Leu
AAT TTA GCA GAT AGA ATG CTT GGA TTG TCT GGA GTG CAG GAA ATT AAG GAA CAG GGT GTT GGA CTG ATA GCA GAG TGT AGA ACT TTC TTG GAT TCT ATT GCT GGC ACT TTG (3730)

1000                                    1010                                    1020                                    1030
Lys Ser Met Met Phe Gly Phe His His Ser Val Thr Val Glu Ile Ile Asn Thr Val Leu Cys Phe Val Lys Ser Gly Ile Leu Leu Tyr Val Ile Gln Gln Leu Asn Gln
AAA TCT ATG ATG TTT GGC TTT CAT CAT TCT GTG ACT GTT GAA ATT ATA AAT ACT GTG CTT TGT TTT GTT AAG AGT GGA ATC CTG CTT TAT GTC ATA CAA CAA TTG AAC CAA (3841)

            1040                                    1050                                    1060                                    1070
Asp Glu His Ser His Ile Ile Gly Leu Leu Arg Val Met Asn Tyr Ala Asp Ile Gly Cys Ser Val Ile Ser Cys Gly Lys Val Phe Ser Lys Met Leu Glu Thr Val Phe
GAT GAA CAC TCT CAC ATA ATT GGT TTG TTC AGA GTT ATG AAT TAT GCA GAT ATT GGC TGT TCA GTT ATT TCA TGT GGT AAA GTT TTT TCC AAA ATG TTA GAA ACA GTT TTT (3952)

            2C
                        1080                                    1090                                    1100                                    1110
Asn Trp Gln Met Asp Ser Arg Met Met Glu Leu Arg Thr Gln Ser Phe Ser Asn Trp Leu Arg Asp Ile Cys Ser Gly Ile Thr Ile Phe Lys Ser Phe Lys Asp Ala Ile
AAT TGG CAA ATG GAT TCT AGA ATG ATG GAG CTC AGG ACT CAG AGC TTC TCT AAT TGG TTA AGA GAT ATT TGT TCA GGA ATT ACT ATT TTT AAA AGT TTT AAG GAT GCC ATA (4063)

                                    1120                                    1130                                    1140
Tyr Trp Leu Tyr Thr Lys Leu Lys Asp Phe Tyr Glu Val Asn Tyr Gly Lys Lys Lys Asp Ile Leu Asn Ile Leu Lys Asp Asn Gln Gln Lys Ile Glu Lys Ala Ile Glu
TAT TGG TTA TAT ACA AAA TTG AAG GAT TTT TAT GAA GTA AAT TAT GGC AAG AAA AAG GAT ATT CTT AAT ATT CTC AAA GAT AAT CAG CAA AAA ATA GAA AAA GCC ATT GAA (4174)

        1150                                    1160                                    1170                                    1180
Glu Ala Asp Asn Phe Cys Ile Leu Gln Ile Gln Asp Val Glu Lys Phe Asp Gln Tyr Gln Lys Gly Val Asp Leu Ile Gln Lys Leu Arg Thr Val His Ser Met Ala Gln
GAA GCA GAC AAT TTT TGC ATT TTG CAA ATT CAA GAT GTA GAG AAA TTT GAT CAG TAT CAG AAA GGG GTT GAT TTA ATA CAA AAG CTG AGA ACT GTC CAT TCA ATG GCG CAA (4285)

                    1190                                    1200                                    1210                                    1220
Val Asp Pro Asn Leu Gly Val His Leu Ser Pro Leu Arg Asp Cys Ile Ala Arg Val His Gln Lys Leu Lys Asn Leu Gly Ser Ile Asn Gln Ala Met Val Thr Arg Cys
GTT GAC CCC AAT TTG GGC GTT CAT TTG TCA CCT CTC AGA GAT TGC ATA GCA AGA GTC CAC CAA AAG CTC AAG AAT CTT GGA TCT ATA AAT CAG GCC ATG GTA ACA AGA TGT (4396)

                                1230                                    1240                                    1250
Glu Pro Val Val Cys Tyr Leu Tyr Gly Lys Arg Gly Gly Gly Lys Ser Leu Thr Ser Ile Ala Leu Ala Thr Lys Ile Cys Lys His Tyr Gly Val Glu Pro Glu Lys Asn
GAG CCA GTT GTT TGC TAT TTG TAT GGC AAA AGA GGG GGA GGG AAA AGC TTG ACT TCA ATT GCA TTG GCA ACC AAA ATT TGT AAA CAC TAT GGT GTT GAA CCT GAG AAA AAT (4507)

    1260                                    1270                                    1280                                    1290
Ile Tyr Thr Lys Pro Val Ala Ser Asp Tyr Trp Asp Gly Tyr Ser Gly Gln Leu Val Cys Ile Ile Asp Asp Ile Gly Gln Asn Thr Thr Asp Glu Asp Trp Ser Asp Phe
ATT TAC ACC AAA CCT GTG GCC TCA GAT TAT TGG GAT GGA TAT AGT GGA CAA TTA GTT TGC ATT ATT GAT GAT ATT GGC CAA AAC ACA ACA GAT GAA GAT TGG TCA GAT TTT (4618)

                1300                                    1310                                    1320                                    1330
Cys Gln Leu Val Ser Gly Cys Pro Met Arg Leu Asn Met Ala Ser Leu Glu Glu Lys Gly Arg His Phe Ser Ser Pro Phe Ile Ile Ala Thr Ser Asn Trp Ser Asn Pro
TGT CAA TTA GTG TCA GGA TGC CCA ATG AGA TTG AAT ATG GCT TCT CTA GAG GAG AAG GGC AGA CAT TTT TCC TCT CCT TTT ATA ATA GCA ACT TCA AAT TGG TCA AAT CCA (4729)

                            1340                                    1350                                    1360
Ser Pro Lys Thr Val Tyr Val Lys Glu Ala Ile Asp Arg Arg Leu His Phe Lys Val Glu Val Lys Pro Ala Ser Phe Phe Lys Asn Pro His Asn Asp Met Leu Asn Val
ACT CCA AAA ACA GTT TAT GTT AAG GAA GCA ATT GAT CGT AGG CTT CAT TTT AAG GTT GAA GTT AAA CCT GCT TCA TTT TTT AAA AAT CCT CAC AAT GAT ATG TTG AAT GTT (4840)

1370                                    1380                                    1390                                    1400
Asn Leu Ala Lys Thr Asn Asp Ala Ile Lys Asp Met Ser Cys Val Asp Leu Ile Met Asp Gly His Asn Ile Ser Leu Met Asp Leu Leu Ser Ser Leu Val Met Thr Val
AAT TTC GCC AAA ACA AAT GAT GCA ATT AAG GAC ATG TCT TGT GTT GAT TTA ATA ATG GAT GGA CAC AAT ATT TCA TTG ATG GAT TTA CTT AGT TCC TTA GTG ATG ACA GTT (4951)

                                                                P3,3A
            1410                                    1420                                    1430                                    1440
Glu Ile Arg Lys Gln Asn Met Ser Glu Phe Met Glu Leu Trp Ser Gln Gly Ile Ser Asp Asp Asp Asn Asp Ser Ala Val Ala Glu Phe Phe Gln Ser Phe Pro Ser Gly
GAA ATT AGG AAA CAG AAT ATG AGT GAA TTC ATG GAG TTG TGG TCT CAG GGA ATT TCA GAT GAT GAC AAT GAT AGT GCA GTG GCT GAG TTT TTC CAG TCT TTT CCA TCT GGT (5062)

                        1450                                    1460                                    1470                                    1480
Glu Pro Ser Asn Trp Lys Leu Ser Ser Phe Phe Gln Ser Val Thr Asn His Lys Trp Val Ala Val Gly Ala Ala Val Gly Ile Leu Gly Val Leu Val Gly Gly Trp Phe
GAA CCA TCA AAT TGG AAG TTA TCT AGT TTT TTC CAA TCT GTC ACT AAT CAC AAG TGG GTT GCT GTG GGA GCT GCA GTT GGC ATT CTT GGA GTG CTT GTG GGA GGA TGG TTT (5173)

                3B                                                                                          3C
                                    1490                                    1500                                    1510
Val Tyr Lys His Phe Ser Arg Lys Glu Glu Glu Pro Ile Pro Ala Glu Gly Val Tyr His Gly Val Thr Lys Pro Lys Gln Val Ile Lys Leu Asp Ala Asp Pro Val Glu
GTG TAT AAG CAT TTT TCC CGC AAA GAG GAA GAA CCA ATT CCA GCT GAA GGG GTT TAT CAT GGC GTG ACT AAG CCC AAA CAA GTG ATT AAA TTC GAT GCA GAT CCA GTA GAG (5284)
```

FIGURE 1b

```
1520                                    1530                                    1540                                    1550
Ser Gln Ser Thr Leu Glu Ile Ala Gly Leu Val Arg Lys Asn Leu Val Gln Phe Gly Val Gly Glu Lys Asn Gly Cys Val Arg Trp Val Met Asn Ala Leu Gly Val Lys
TCC CAG TCA ACT CTA GAA ATA GCA GGA TTA GTT AGG AAA AAT CTG GTT CAG TTT GGA GTT GGT GAG AAA AAT GGA TGT GTG AGA TGG GTC ATG AAT GCC TTA GGA GTG AAG (5395)

1560                                    1570                                    1580                                    1590
Asp Asp Trp Leu Leu Val Pro Ser His Ala Tyr Lys Phe Glu Lys Asp Tyr Glu Met Met Glu Phe Tyr Phe Asn Arg Gly Gly Thr Tyr Tyr Ser Ile Ser Ala Gly Asn
GAT GAT TGG TTG TTA GTA CCT TCT CAT GCT TAT AAA TTT GAA AAG GAT TAT GAA ATG ATG GAG TTT TAC TTC AAT AGA GGT GGA ACT TAC TAT TCA ATT TCA GCT GGT AAT (5506)

1600                                    1610                                    1620
Val Val Ile Gln Ser Leu Asp Val Gly Phe Gln Asp Val Val Leu Met Lys Val Pro Thr Ile Pro Lys Phe Arg Asp Ile Thr Gln His Phe Ile Lys Lys Gly Asp Val
GTT GTT ATT CAA TCT TTA GAT GTG GGA TTT CAA GAT GTT GTT TTA ATG AAG GTT CCT ACA ATT CCC AAG TTT AGA GAT ATT ACT CAA CAC TTT ATT AAG AAA GGA GAT GTG (5617)

1630                                    1640                                    1650                                    1660
Pro Arg Ala Leu Asn Arg Leu Ala Thr Leu Val Thr Thr Val Asn Gly Thr Pro Met Leu Ile Ser Glu Gly Pro Leu Lys Met Glu Glu Lys Ala Thr Tyr Val His Lys
CCT AGA GCC TTA AAT CGC TTG GCA ACA TTA GTG ACA ACC GTT AAT GGA ACT CCT ATG TTA ATT TCT GAG GGA CCA TTA AAG ATG GAA GAA AAA GCC ACT TAT GTT CAT AAG (5728)

1670                                    3D 1680                                 1690                                    1700
Lys Asn Asp Gly Thr Thr Val Asp Leu Thr Val Asp Gln Ala Trp Arg Gly Lys Gly Glu Gly Leu Pro Gly Met Cys Gly Gly Ala Leu Val Ser Ser Asn Gln Ser Ile
AAG AAT GAT GGT ACT ACA GTT GAT TTC ACT GTA GAT CAG GCA TGG AGA GGA AAA GGT GAA GGT CTT CCT GGA ATG TGT GGT GGG GCC CTA GTG TCA TCA AAT CAG TCC ATA (5839)

1710                                    1720                                    1730
Gln Asn Ala Ile Leu Gly Ile His Val Ala Gly Gly Asn Ser Ile Leu Val Ala Lys Leu Val Thr Gln Glu Met Phe Gln Asn Ile Asp Lys Lys Ile Glu Ser Gln Arg
CAG AAT GCA ATT TTG GGT ATT CAT GTT GCT GGA GGA AAT TCA ATT CTT GTG GCA AAG CTG GTT ACT CAA GAA ATG TTT CAA AAC ATT GAT AAG AAA ATT GAA AGT CAG AGA (5940)

1740                                    1750                                    1760                                    1770
Ile Met Lys Val Glu Phe Thr Gln Cys Ser Met Asn Val Val Ser Lys Thr Leu Phe Arg Lys Ser Pro Ile His His His Ile Asp Lys Thr Met Ile Asn Phe Pro Ala
ATA ATG AAA GTG GAA TTT ACT CAA TGT TCA ATG AAT GTA GTC TCC AAA ACG CTT TTT AGA AAG AGT CCC ATT CAT CAC CAC ATT GAT AAA ACC ATG ATT AAT TTT CCT GCA (6051)

1780                                    1790                                    1800                                    1810
Ala Met Pro Phe Ser Lys Ala Glu Ile Asp Pro Met Ala Met Met Leu Ser Lys Tyr Ser Leu Pro Ile Val Glu Glu Pro Glu Asp Tyr Lys Glu Ala Ser Val Phe Tyr
GCT ATG CCT TTC TCT AAA GCT GAA ATT GAT CCA ATG GCT ATG ATG TTG TCC AAA TAT TCA TTA CCT ATT GTG GAG GAA CCA GAG GAT TAC AAG GAA GCT TCA GTT TTT TAT (6162)

1820                                    1830                                    1840                                    1850
Gln Asn Lys Ile Val Gly Lys Thr Gln Leu Val Asp Asp Phe Leu Asp Leu Asp Met Ala Ile Thr Gly Ala Pro Gly Ile Asp Ala Ile Asn Met Asp Ser Ser Pro Gly
CAA AAC AAA ATA GTA GGC AAG ACT CAG CTA GTT GAT GAC TTT TTA GAT CTT GAT ATG GCT ATT ACA GGG GCT CCA GGC ATT GAT GCT ATC AAT ATG GAT TCA TCT CCT GGG (6273)

1860                                    1870                                    1880
Phe Pro Tyr Val Gln Glu Lys Leu Thr Lys Arg Asp Leu Ile Trp Leu Asp Glu Asn Gly Leu Leu Leu Gly Val His Pro Arg Leu Ala Gln Arg Ile Leu Phe Asn Thr
TTT CCT TAT GTT CAA GAA AAA TTG ACC AAA AGA GAT TTA ATT TGG TTG GAT GAA AAT GGT TTG CTG TTA GGA GTT CAC CCA AGA TTG GCC CAG AGA ATT TTA TTT AAT ACT (6384)

1890                                    1900                                    1910                                    1920
Val Met Met Glu Asn Cys Ser Asp Leu Asp Val Val Phe Thr Thr Cys Pro Lys Asp Glu Leu Arg Pro Leu Glu Lys Val Leu Glu Ser Lys Thr Arg Ala Ile Asp Ala
GTC ATG ATG GAA AAT TGT TCT GAC TTA GAT GTT GTT TTT ACA ACT TGT CCA AAA GAT GAA TTG AGA CCA TTA GAG AAA GTT TTG GAA TCA AAA ACA AGA GCC ATT GAT GCT (6495)

1930                                    1940                                    1950                                    1960
Cys Pro Leu Asp Tyr Thr Ile Leu Cys Arg Met Tyr Trp Gly Pro Ala Ile Ser Tyr Phe His Leu Asn Pro Gly Phe His Thr Gly Val Ala Ile Gly Ile Asp Pro Asp
TGT CCT TTG GAT TAT ACA ATT CTA TGT CGA ATG TAT TGG GGT CCA GCT ATC AGT TAT TTC CAT TTG AAT CCA GGG TTT CAC ACA GGT GTT GCT ATT GGC ATA GAT CCT GAT (6606)

1970                                    1980                                    1990
Arg Gln Trp Asp Glu Leu Phe Lys Thr Met Ile Arg Phe Gly Asp Val Gly Leu Asp Leu Asp Phe Ser Ala Phe Asp Ala Ser Leu Ser Pro Phe Met Ile Arg Glu Ala
AGA CAG TGG GAT GAA TTA TTT AAA ACA ATG ATA AGA TTT GGA GAT GTT GGT CTT GAT TTA GAT TTC TCT GCT TTT GAT GCC AGT CTT AGT CCA TTT ATG ATT AGG GAA GCA (6717)

2000                                    2010                                    2020                                    2030
Gly Arg Ile Met Ser Glu Leu Ser Gly Thr Pro Ser His Phe Gly Thr Ala Leu Ile Asn Thr Ile Ile Tyr Ser Lys His Leu Leu Tyr Asn Cys Cys Tyr His Val Cys
GGT AGA ATC ATG AGT GAA TTA TCT GGA ACA CCA TCT CAT TTT GGA ACA GCT CTT ATC AAT ACT ATC ATT TAT TCT AAA CAT CTG CTG TAC AAC TGT TGT TAT CAT GTT TGT (6828)

2040                                    2050                                    2060                                    2070
Gly Ser Met Pro Ser Gly Ser Pro Cys Thr Ala Leu Leu Asn Ser Ile Ile Asn Asn Ile Asn Leu Tyr Tyr Val Phe Ser Lys Ile Phe Gly Lys Ser Pro Val Phe Phe
GGT TCA ATG CCT TCT GGG TCT CCT TGC ACA GCT TTG TTG AAT TCA ATT ATT AAT AAT ATT AAT CTG TAT TAT GTG TTT TCT AAA ATA TTT GGA AAG TCT CCA GTT TTC TTT (6939)

2080                                    2090                                    2100
Cys Gln Ala Leu Arg Ile Leu Cys Tyr Gly Asp Asp Val Leu Ile Val Phe Ser Arg Asp Val Gln Ile Asp Asn Leu Asp Leu Ile Gly Gln Lys Ile Val Asp Glu Phe
TGT CAA GCT TTG AGG ATC CTT TGT TAC GGA GAT GAT GTT TTG ATA GTT TTT TCC AGA GAT GTT CAA ATT GAC AAT CTT GAC TTG ATT GGA CAG AAA ATT GTA GAT GAG TTC (7050)

2110                                    2120                                    2130                                    2140
Lys Lys Leu Gly Met Thr Ala Thr Ser Ala Asp Lys Asn Val Pro Gln Leu Lys Pro Val Ser Glu Leu Thr Phe Leu Lys Arg Ser Phe Asn Leu Val Glu Asp Arg Ile
AAA AAA CTT GGC ATG ACA GCC ACC TCA GCT GAT AAA AAT GTG CCT CAA CTG AAG CCA GTT TCA GAA TTG ACT TTT CTC AAA AGA TCT TTC AAT TTG GTG GAG GAT AGA ATT (7161)

2150                                    2160                                    2170                                    2180
Arg Pro Ala Ile Ser Glu Lys Thr Ile Trp Ser Leu Met Ala Trp Gln Arg Ser Asn Ala Glu Phe Glu Gln Asn Leu Glu Asn Ala Gln Trp Phe Ala Phe Met His Gly
AGA CCT GCA ATT TCA GAA AAG ACA ATT TGG TCT TTG ATG GCT TGG CAG AGA AGT AAC GCT GAG TTT GAG CAG AAT TTA GAA AAT GCT CAG TGG TTT GCT TTT ATG CAT GGC (7272)

2190                                    2200                                    2210                                    2220
Tyr Glu Phe Tyr Gln Lys Phe Tyr Tyr Phe Val Gln Ser Cys Leu Glu Lys Glu Met Ile Glu Tyr Arg Leu Lys Ser Tyr Asp Trp Trp Arg Met Arg Phe Tyr Asp Gln
TAT GAG TTC TAT CAG AAA TTT TAT TAT TTT GTT CAG TCC TGT TTG GAG AAA GAG ATG ATA GAA TAT AGA CTT AAA TCT TAT GAT TGG TGG AGA ATG AGA TTT TAT GAC CAG (7383)

2227
Cys Phe Ile Cys Asp Leu Ser OP
TGT TTC ATT TGT GAC CTT TCA TGA TTTGTTTAAACAAATTTTCTTACTCTTTCTGAGGTTTGTTTATTTCTTTTGTCCCCTAACTAAAAAAAAAAAAAAA (7484)

Translated Mol. Weight = 251,940
```

FIGURE 1c

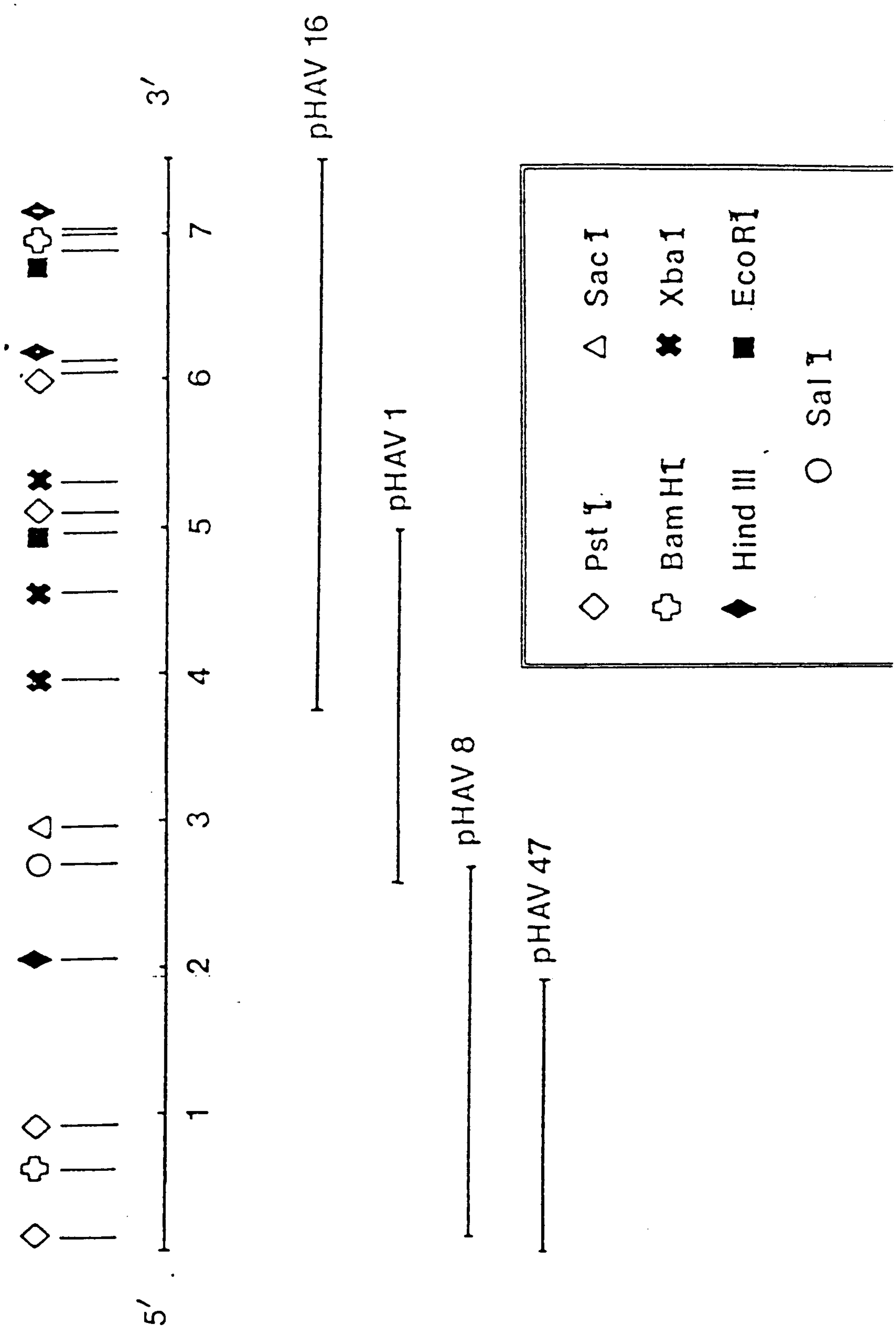
5′
3′
1
2
3
4
5
6
7
pHAV 16
pHAV 1
pHAV 8
pHAV 47
Pst I
Sac I
Bam HI
Xba I
Hind III
EcoRI
Sal I

FIGURE 2

Figure 3: SCHAEMATIC OF STRUCTURE OF HAV EXPRESSION PLASMIDS

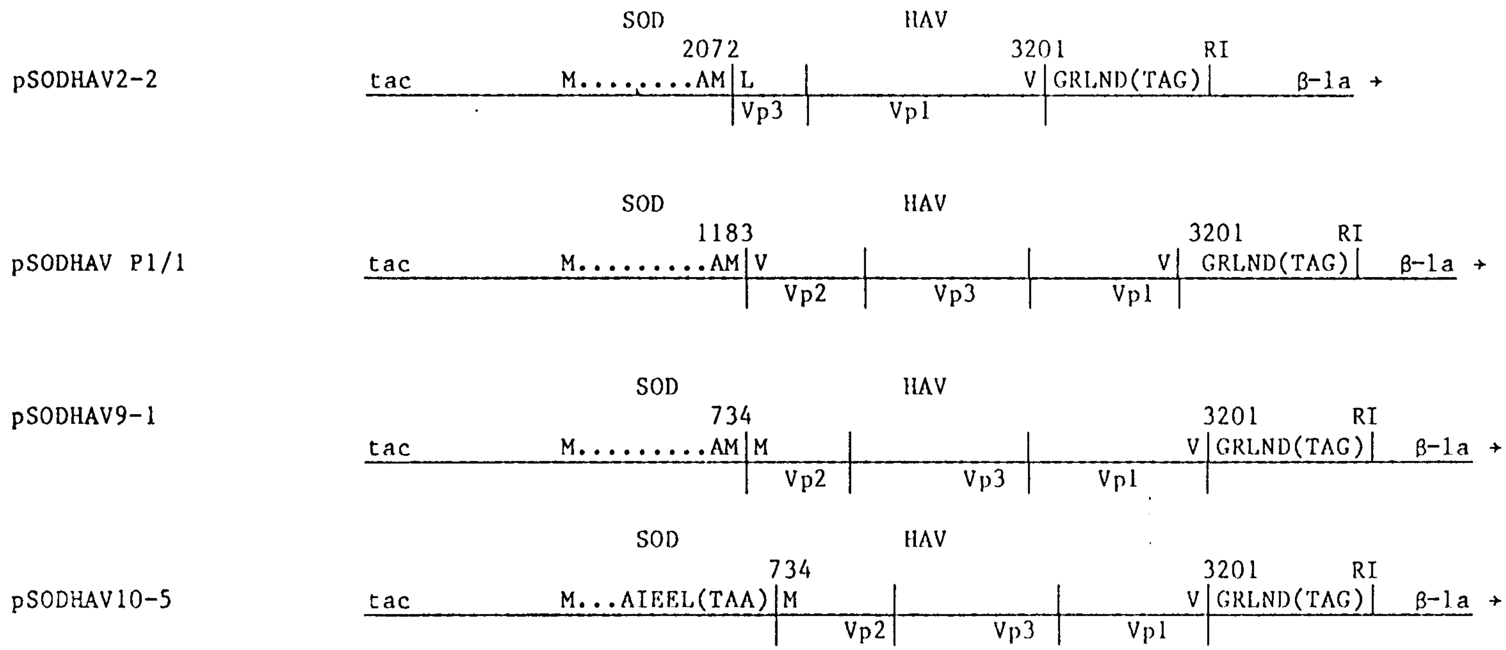


tac= trp/lac promoter/operator.
(TAA), (TAG)= stop codons.
NUMBERS give HAV nucleotides at beginning and end of HAV sequence.
CAPITAL LETTERS give amino acid residues (one letter code) at start/stops and SOD/HAV/linker junctions.
RI: EcoRI restriction site.

FIGURE 3

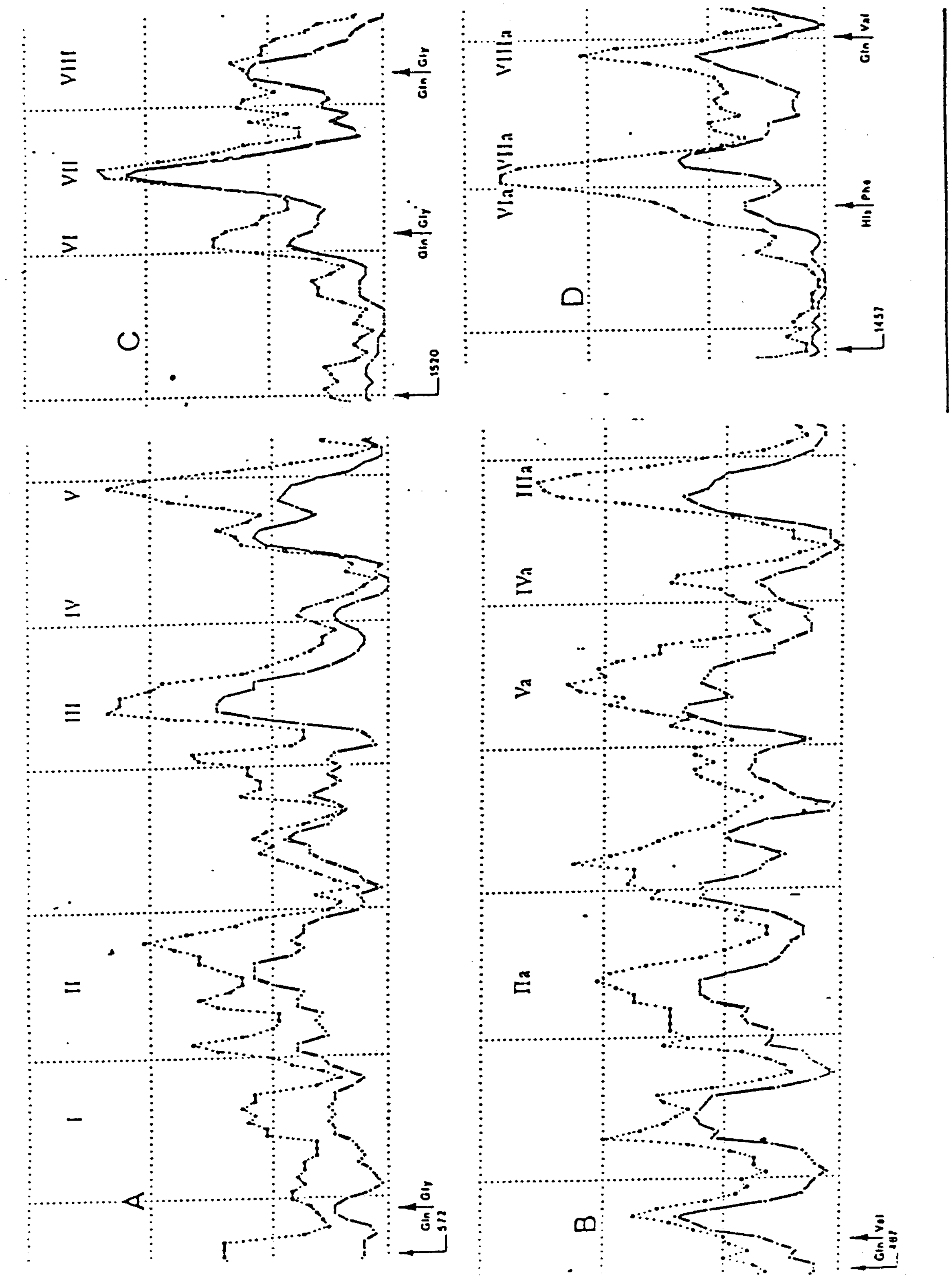
A
I
II
III
IV
V
Gln Gly
572
B
IIa
Va
IVa
IIIa
Gln Val
487
C
VI
VII
VIII
Gln Gly
Gln Gly
1520
D
VIa
VIIa
VIIIa
His Phe
Gln Val
1457

FIGURE 4

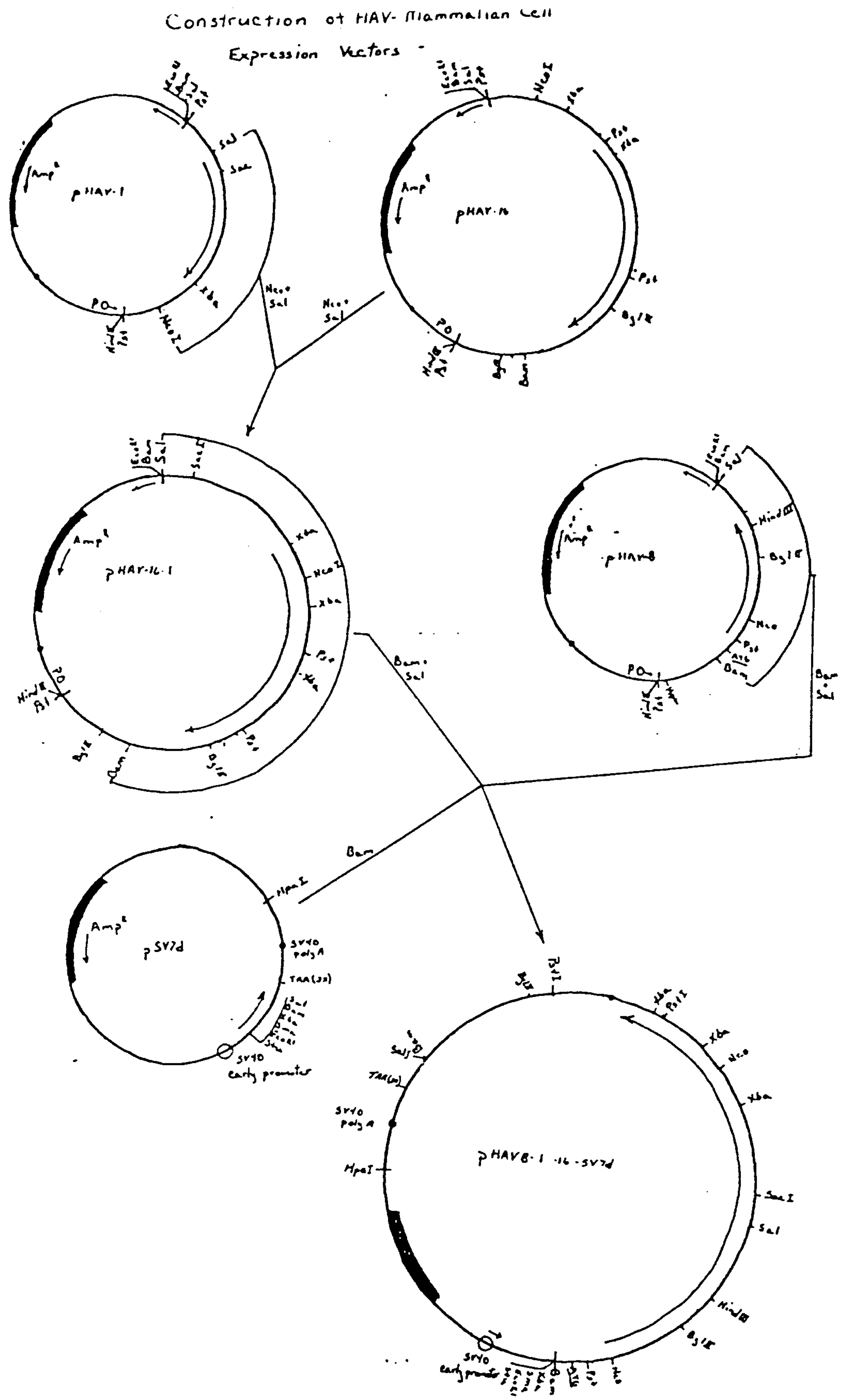
Construction of HAV-Mammalian Cell Expression Vectors -
pHAV-1
pHAV-16
pHAV-16-1
pHAVB
pSV7d
pHAVB-1-16-SV7d
Amp^R
SV40 early promoter
SV40 polyA

FIGURE 5

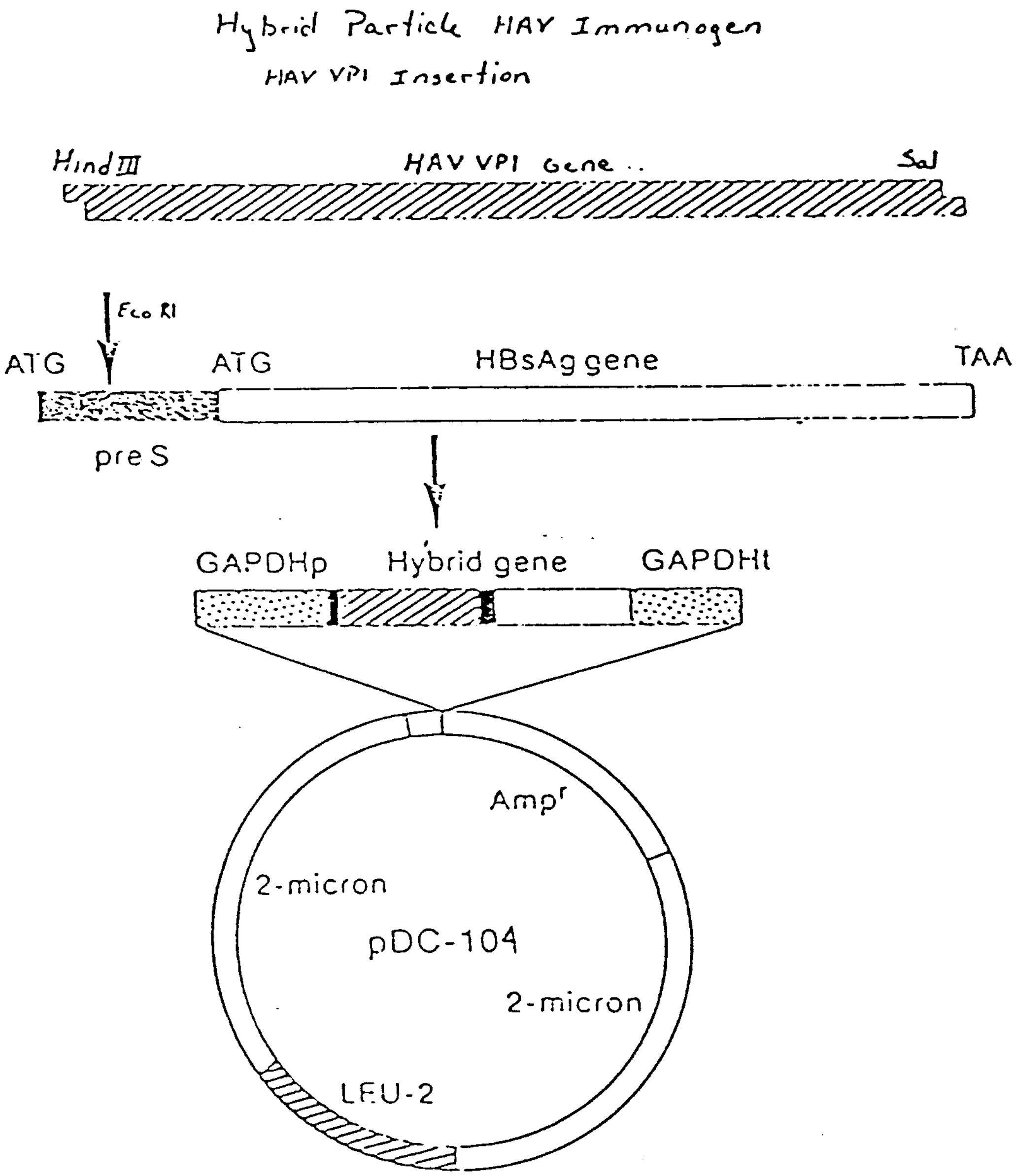
Hybrid Particle HAV Immunogen
HAV VP1 Insertion
HindIII
HAV VP1 Gene ..
Sal
EcoRI
ATG
ATG
HBsAg gene
TAA
pre S
GAPDHp
Hybrid gene
GAPDHt
Amp^r
2-micron
pDC-104
2-micron
LEU-2

FIGURE 6

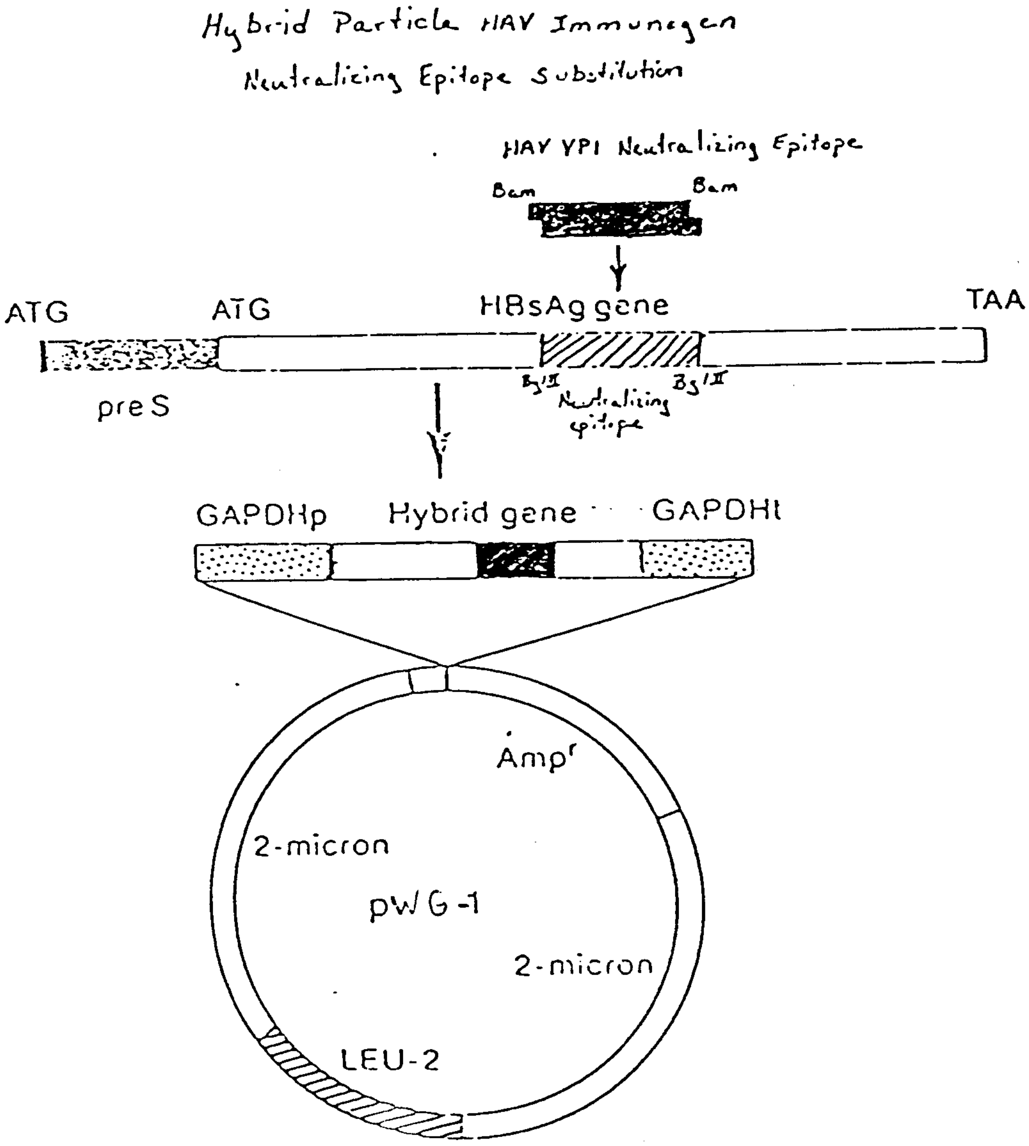
Hybrid Particle HAV Immunogen
Neutralizing Epitope Substitution
HAV YP1 Neutralizing Epitope
Bam
Bam
ATG
ATG
HBsAg gene
TAA
preS
BglII
BglII
Neutralizing epitope
GAPDHp
Hybrid gene
GAPDHt
Amp^r
2-micron
pWG-1
2-micron
LEU-2

FIGURE 7